# EUROPEAN PATENT SPECIFICATION

(11) **EP 0 411 038 B1**
(45) Date of publication and mention of the grant of the patent: **28.12.1994**
(21) Application number: 89905521.4
(22) Date of filing: 21.04.1989
(51) Int. Cl.: G01N 33/549, C12Q 1/00, C12N 11/04, C12Q 1/24, C12Q 1/18

(54) **PROCESS FOR FORMING AND USING MICRODROPLETS**
VERFAHREN ZUM FORMEN UND ZUM GEBRAUCH VON MIKROTRÖPCHEN
PROCEDE POUR FORMER ET POUR UTILISER DES MICROGOUTTES

(30) Priority: 22.04.1988 US 184968; 22.04.1988 US 184969; 22.04.1988 US 185083; 22.04.1988 US 185084; 22.04.1988 US 185136; 22.04.1988 US 185156; 22.04.1988 US 185160; 22.04.1988 US 185475
(43) Date of publication of application: 06.02.1991
(73) Proprietor: MASSACHUSETTS INSTITUTE OF TECHNOLOGY, Cambridge, MA 02139 (US)
(72) Inventor: WEAVER, James, C., Sudbury, MA 01776 (US); WILLIAMS, Gregory, B., Tewksbury, MA 01876 (US); BLISS, Jonathan, G., Somerville, MA 02143 (US); POWELL, Kevin, T., Boston, MA 02114 (US); HARRISON, Gail, I., Watertown, MA 02172 (US); JOSEPH, Julian, Cedarhurst, NY 11516 (US)
(74) Representative: Holdcroft, James Gerald, Dr.
(86) International application number: US8901699
(87) International publication number: WO8910566

(56) References cited:
- EP-A- 0 109 861
- EP-A- 0 114 756
- WO-A-82/02561
- US-A- 4 649 109
- Ann. N.Y. Acad. Sci., vol. 501, 1987, G.B. Williams et al., pp. 350-353
- Biotechnology & Bioengineering Symp., no. 17, 1986, John Wiley & Sons Inc.,J.C. Weaver, pp. 185-195

## Description

### Background of the Invention

Measurement or isolation of desirable cells is a well established goal of much of industrial microbiology and biotechnology (see, for example, Queener and Lively in *Manual of Industrial Microbiology and Biotechnology*, Demain and Solomon (Eds.), American Soc. Microbiol., Washington, D.C., 155 - 169, 1986; White et al, ibid, pp. 24 - 31). Selection strategies, which are based on preferential growth and recovery of desired cells, are often possible. For example, selection can be accomplished by genetically manipulating cells such that a desired trait is coupled with resistance to antibiotics or to the ability to grow in the absence of certain compounds. However, there are many cases wherein selection does not work well or does not work at all. In such instances cells are often isolated by screening, i.e. by methods wherein cells are cloned, identified and one or more desirable properties measured, such that a particular clone is identified, then physically removed, and thereby isolated.

One well established application of screening methods for microorganisms involves cells which have been exposed to mutagenic conditions, or to genetic splicing procedures, such that a large population of cells results, but of which only a very small number of cells are successes, e.g. cells which both grow and produce a desired molecule at a high level. Plating of such cells on petri dishes such that separated colonies arise is then followed by chemical assays, such that, for example, a successful colony which is scored postive (+) by use of radioimmunoassay, or is colored through use of a colorimetric indicator. The number of cells in such colonies is typically large. For example, in the case of cells which are microorganisms such as bacteria, such colonies typically contain between 10⁷ and 10⁹ cells. Cells from successful colonies are then removed and suspended in a medium such that the number of cells can be enumerated, and the accumulated amount or the production rate of a desired molecule can be quantitatively determined. This type of procedure requires several steps, and is now generally carried out using relatively macroscopic entities and manual procedures, such as petri dishes (for initial colony formation), visual inspection (for identifying positive colonies), physical manipulation (for colony removal and suspension), physical measurement (for cell enumeration by light scattering or other means) chemical assay (for determination of molecule concentrations at different times) and calculation (for comparison of molecule production rate to known rates).

Screening is often much more difficult when a producing strain already exists, and the task is to seek rare mutants which produce the same molecule, but at higher rates, or to higher final concentrations. In such cases, the first step of scoring positive cells is unnecessary, but then all cells must be quantitatively assayed for relative productivity. This is generally a very time consuming task. Because of the several macroscopic manipulation and assays, a typical screening task can involve formation of many initial colonies and significant manual labor.

Isolation of desirable hybridoma cells which produce and secrete specific monoclonal antibodies (MAbs) at high rates, and to high levels, is another general task. In this general case, once viable hybrid cells have been produced by cell fusion techniques, and selective growth in HAT (hypoxanthine, aminopterin, thymidine) medium has occured for sufficient time that mostly stable hybridomas exist, these hybridomas must be screened for production of a desired Ab (anti-body). Typically this involves more than one use of the screening procedure. Polyclonal samples are grown in microtiter wells (e.g. 1 to 5 ml), which are very large compared to the volumes of individual cells (e.g. V_{cell} ≈ 10⁻¹² ml for bacteria to about 10⁻⁸ ml for mammalian cells). The contents of microtiter wells which exhibit desired activity are diluted sufficiently, so that upon culture in a new set of wells, it is highly probable that a well has zero or one initial cell, so that monoclonal production is obtained, growth is allowed to occur, and the Ab produced within the well is assayed for properties such as specificity and affinity. Although widely used, such procedures are tedious and involve significant manual labor.

Previous methods have been described for isolation of cells with particularly high secretion rates of acetylcholinesterase (AChE), and are based on the use of gel capsules with a semi-permeable barrier which allows entry of specific capturing entities such as inhibitors (I) or antibodies (Ab), but which will not allow the release or outward permeation of the AChE-I or AChE-Ab complex (see Rose, European Patent Application No. 8430036.3, Filed Jan. 20, 1984). However, realistic permeability barriers such as those functioning as dialysis membranes have a broad molecular weight cut-off, and can not provide both significant permeability to Ab and significant retention of AChE-Ab. Such barriers could only function for complexes AChE-I wherein I is a relatively small molecule compared to AChE. For this reason, it is necessary instead to use specific binding sites associated with the gel matrix of a gel microdroplet (GMD), but without a hypothetical semipermeable membrane with presently unrealistic properties.

Another form of isolation process relates to the use of various gel configurations to isolate antibody (Ab) secreting cells, emphasizing the killing of undesired cells by conditioning the regions near desirable cells (see Rose, European Patent Application No. 83307144.2, Filed Nov. 22, 1983), wherein selective coloring or fluorescent tagging of either desired or undesired cells, or areas within the gel in the vacinity of the cells, is described, but there is no teaching of quantitative measurement, or of isolation, based on quantitative determinations of captured Ab.

Methods relating generally to solid phase immunoassays predominantly involves the assay of Ab and Ag (antigen) in a liquid phase or supernatent, and generally teaches that readily washable surfaces are preferred (see, for example, *Alternative Immunoassays*, Wiley, Chichester, 1985; Voller (Eds.) *Immunoassays* *for the 80s*, University Park Press, Baltimore, 1981).

However, the use of Ab-containing gel fragments has been described for measurement of bulk liquid Ag concentrations, wherein macroscopic volumes of gel are prepared with associated Ab, the macroscopic gel is fragmented in order to obtain smaller gel particles, and then contacted with a liquid sample in order to measure Ag present in a liquid sample (see Updike in *Biomedical Applications of* *Immobilized Enzymes and Proteins: Volume 2*, Chang (Ed.), 71 - 86, 1977).

Prior use of GMDs for measurement is based on the extracellular accumulation of solubilized metabolites, such as metabolic acids, within the small volume of a GMD which is coated with a membrane material, or which is surrounded by a another liquid such as mineral oil (see U.S. Patents No; 4,401,755 and 4,643,968 by Weaver, whose teachings are hereby incorporated by reference, and also Weaver et al., Ann. N.Y. Acad. Sci., 434: 363 - 372, 1984; Weaver, Biotech. and Bioengr. Symp. 17, 185 - 195, 1986; Williams et al., Ann. N. Y. Acad. Sci., 501: 350 - 353, 1987).

Prior use of GMDs for isolation is based on the use of lysing means to release constituent molecules from the interior of a cell, followed, for example, by provision of soluble Ab in order to form immunoprecipitates which are retained by means of entrapment within the gel matrix of the GMDs. Such immunoprecipitates can then be further reacted with optically labeled Ab or Ag prior to the use of a flow cytometer/cell sorter. It is additionally described that such immunoprecipitates can be labeled with magnetic molecules prior to using magnetic forces to physically isolate GMDs. However, this prior use does not teach the use of pre-existing binding sites within GMDs as the basis of capture of secreted or released molecules, but instead only describes the use of soluble Ab and Ag, and the like, for forming immunoprecipitates within the gel matrix of a GMD (see U.S. Patent No. 4,399,219 by Weaver, the teachings of which are incorporated herein by reference, and also Weaver, Biotech. and Bioengr. Symp. 17, 185 - 195, 1986).

Measurement and isolation of biological entities such as cells, virus, nucleic acids and antibody-antigen complexes is a well established goal in pure and applied biology, with many important applications in fields such as clincial microbiology, cancer diagnosis and treatment, environmental science, food safety, toxicology, and research and development in basic and applied biology. Although a significant number of assays and tests are directed towards virus, nucleic acids and antibody-antigen complexes, most well established methods for determining measurements of such biological entities relate to measurements on cells. For this reason, the main features of prior methods for measuring and isolating biological entities are illustrated by considering prior methods for making measurements on cells.

Similarly, prior methods for determining growth of viruses involve the use of cell culture, such that sample viruses infect one or more provided cells, and grow within the infected cells, often resulting in cell lysis and releasing a large number of viruses. Prior methods for determining growth of chlamydia also involve growth within host cells.

Similarly, prior methods for determining the effects of compounds and agents on the growth of viruses involve the use of cell culture, such that sample viruses infect one or more provided cells, and grow within the infected cells, often resulting in cell lysis and releasing a large number of viruses. Prior methods for determining growth of chlamydia also involve growth within host cells.

Further, prior methods for determining growth of nucleic acids involve a cyclic incubation which results in a doubling of specific nucleic acids with each cycle (see Mullis et al., U.S. Patent 4,683,195). Finally, prior methods for determining growth of immunological complexes such as antibody-antigen complexes are carried out in a homogeneous phase wherein the reactants are in solution, or using a solid phase, wherein one or more reactants are attached to a solid surface.

Prior cell analysis methods involve two major classes of assays. The first class rapidly detects and identifies specific cells directly from a primary sample, but does not determine cell viability. The most widely used in this class are specific ligand binding assays, e.g. immunoassays and genetic probes. However, they require many cells, and do not distinguish between dead and viable cells. This restricts their use to samples in which sufficient numbers of cells are present, and to determinations in which direct assessment of the physiological state of the cell is irrelevant. The second class of assays is used for viable cell measurements either directly using the primary sample, or using a subculture of the primary sample. The most traditional and widely used method is the plate count, which allows determination of single cell viability, based on growth, under many test conditions. An important attribute of viable plate enumeration is that time required to obtain a determination is independent of the concentration of the cell in the sample, because formation of each colony proceeds from an initial single cell. The major disadvantage is its slowness, as typical determinations require one-half to several days, and are also labor- and materials-intensive.

The disadvantages of viable plating can better be appreciated by drawing attention to its basic attributes. Viable plating is a well established, important method for qualitatively determining the growth of cells, particularly the presence or absence of growth for given conditions, and is often based on the growth of initial cells into distinct colonies. Viable plating typically involves the spreading of a suspension of cells onto the surface of a gel-containing petri dish, with or without the pouring of a gel layer over the first gel surface. The gels are provided with nutrients, such that following an incubation period at a suitable temperature, many generations of growth occur, which leads to formation of visible colonies. For many microorganisms formation of visible colonies requires growth for 22 to 30 generations and therefore produces colonies containing 10⁷ to 10⁹ cells. (See Sharpe, in *Mechanizing Microbiology,* A. N. Sharpe and D. S. Clark (Eds.) Charles C. Thomas, Springfield, 19 - 40, 1978). Although conventional viable plating leads to formation of colonies, and thereby provides a basis for counting viable cells by counting colonies, the presence or absence of colonies only allows an inference that the conditions present in the gel support do or do not support growth. For this reason, conventional viable plating is not well suited to quantitative determinations such as cell growth rate and lag time, because viable plating based on visual inspection counts the number of colonies formed, but does not determine how the cellular material or amount of cellular constituents in the colonies varies with time. An additional complication arises because the nutrient and metabolite concentrations within a colony comprise a microenvironment, which generally changes with time in a variable way as microcolonies increase to form larger colonies with many cells in close proximity. The microenvironment within a large colony can also have significant heterogeneity of chemical composition within the microcolony, so that different cells within a large colony experience different growth conditions. Further, although some methods are based on a straightforward extension and application of scanning optical methods for determination of optical properties of colonies on or in gel slabs, such methods suffer from relatively large cost and size, and, because of the relatively large gel slab size, do not allow incubation conditions to be changed rapidly at the site of the cells within the gel. (See Glaser in *New Approaches to* *the Identification of Microorganisms Proceedings of a Symposium on Rapid* *Methods and Automation in Microbiology*, C.-G. Heden and T. Illeni (Eds.), Wiley, New York, 3 - 12, 1975).

Instrumented methods for rapidly determining cell or culture growth and/or metabolic activity have been developed which only partially address the limitations of the viable plate assay. These include optical techniques for growth determination such as those which measure the change in light scattering due to many cells in a liquid suspended culture (See, for example, Edberg and Berger, in *Rapid Methods and Automation in Microbiology and Immunology*, K. O. HabermehI, Ed., Springer-Verlag, Berlin, 215 - 221, 1985), and a variety of metabolic activity based techniques which measure changes due to many cells in an analyzed sample. Examples include changes in extracellular pH (See, for example, *Cowan and Steel 's Manual for the Identification of Medical Bacteria*, Cambridge University Press, Cambridge, 1974; *Manual of Methods for General Bac**teriology*, P. Gerhardt, (Ed), American Society for Microbiology, Washington, 1981), carbon dioxide release (see, for example, Courcol et al., J. Clin. Microbiol., 25: 26 - 29, 1986; Manca et al., J. Clin. Microbiol., 23: 401 - 403, 1986), electrical impedance (see, for example, Stewart, J. Exp. Med., 4: 235 - 245, 1899; Eden and Eden *Impedance Microbiology*, Research Studies Press, Letchworth, 1984; Hadley and Yajko, in Instrumental Methods for Rapid Microbiological Analysis, Nelson (Ed.), VCH, Weinheim, 193 - 209, 1985; Bishop and White, J. Food Protect., 49: 739 - 753, 1986), chemiluminescence (see, for example, Neufeld et al., in *Instrumental Methods for Rapid Microbiological Analysis*, Nelson (Ed.), VCH, Weinheim, 51 - 65, 1985; *Rapid Methods and Automation in* *Microbiology and Immunology*, Habermehl (Ed.), Springer-Verlag, Berlin, 1985) or fluorescence (see, for example, Rossi and Warner in *Instrumental Methods for* *Rapid Microbiological Analysis*, Nelson (Ed.), VCH, Weinheim, 1 - 50, 1985; *Rapid Methods and Automation in Microbiology and Immunology*, Habermehl (Ed.), Springer-Verlag, Berlin, 1985). A disadvantage of all such metabolic activity methods is that they are based on combined effects of a large number of cells, and therefore generally require an initial process, based on plating, to obtain initial colonies for purposes of inoculation of the analyzed sample, such that the determinations based on many cells at least are based on a monopopulation, i.e. a population comprised nominally of the same type of cells. For this reason, although a total population cell determination may itself be rapid, it is generally preceeded by a viable plating method, or its equivalent, which is slow. Thus, the total analysis time, counted from receipt of a primary or initial sample to a cell growth determination, is the sum of both, and therefore still long.

Further, because such determinations are based on the combined effect of a large, but unkown number, of cells, such total population determinations do not actually yield a count. In contrast, determinations based on many individual measurements, each associated with an initial single cell, can yield a count.

Finally, because these total population methods are based on the combined effects of many cells, the time required for a determination becomes significantly longer as the number of cells decreases, i.e. as the sample's cell concentration decreases.

Similarly, prior use of flow cytometry for cell growth measurements (see, for example, Hadley et al. in *Instrumental Methods for Rapid Microbiological* *Analysis*, Nelson (Ed.), VCH, Weinheim, 67 - 89, 1985) is limited, because conventional use of flow cytometry performs measurements on individual cells, or clumps of cells which naturally adhere, in a liquid suspension, and therefore does not have the capability to measure colony formation. For this reason, prior use of flow cytometry can only measure total numbers of cells in a volume in order to determine average growth, and must also, therefore, involve a careful volume measurement, and is dependent on the signal-to-noise ratio of single cell measurements, the signal-to-noise ratio generally being less than satisfactory for many measurements (see, for example, Shapiro *Practical Flow Cytometry*, R. Liss, New York, 1985; Hadley et al. in *Instrumental Methods for Rapid Microbiological* *Analysis*, Nelson (Ed.), VCH, Weinheim, 67 - 89, 1985).

Likewise, quantitative microscopy and image analysis combined with conventional gel preparations, such as gel slabs, petri dishes and the like, although capable of determining colony formation, is tedious in manual versions. Conventional gel slabs, petri dishes and the like cannot provide physical manipulability or a sufficiently fast (small) characteristic diffusion time within the gel, so that cells cannot be rapidly and conveniently exposed to different growth conditions, such as rapid changes in concentrations of nutrients, drugs, hormones, enzymes, antibodies and other chemicals. In addition, conventional gel slabs, petri dishes and the like cannot be readily manipulated physically because of their size, and therefore cannot be readily used for exposure of gel-entrapped cells to *in vivo* conditions. For example, agarose slabs can be used to protect fragile biological entities such as plant protoplasts in cases wherein both free cells and cells entrapped in agarose beads are found to be damaged (see Pasz and Lurquin, BioTechniques 5: 716 - 718, 1987), but such gel slabs have relatively long characteristic diffusion times and cannot be readily inserted into animals in order to provide biological influence. As another example, guiding human cancer chemotherapy by determining human cancer cell growth in immunodeficient mice, which are exposed to trial chemotherapy conditions, can be approached by insertion of a small tissue piece into a mouse (see Bogden, Annal. Chirurgiae Gynaecol., Vol. 74 Suppl. 199, 12 - 27, 1985; Favre et al., Eu. J. Can. Che. Oncol., 22: 1171 - 1178, 1986), but the insertion of conventional gel slabs or petri dishes into the mice would be similarly cumbersome, requiring considerable manual manipulation. For example, slices of clot about 1 to 1.4 mm on a side containing about 1 x 10⁵ to 2 x 10⁵ human cancer cells can be prepared and inserted into a mouse, and subsequent combined growth due to this large number of cells can be determined by measurement of the resulting tumor dimensions. Colony formation is not determined, however, so that such procedures both require manual manipulations and do not result in cell preparations which are readily analyzed quantitatively for cell growth, such as average cell growth rate, or more importantly, the quantitative growth of individual cells (see Fingert, Cancer Res., 47: 3824 - 3829, 1987). Further, in the absence of applied physical forces, transport of molecules within gel materials occurs by diffusion, for which the characteristic diffusion times are dependent on the square of the thickness of the gel slabs or petri dish gel material. These thicknesses are typically 10⁻¹ to 3 x 10⁻¹ cm or larger. For this reason characteristic diffusion times for even small molecules are long, as can be estimated by calculating t_{diffusion} ≈ x²/D, where x is a characteristic dimension or thickness of the gel. The diffusion constant, D, is about 10⁻⁵ cm²/sec for small molecules, and is signficantly smaller, often about 10⁻⁷ cm²/sec for large molecules such as antibodies and enzymes, so that typically t_{diffusion} ≈ 17 to 50 minutes for small molecules, and about 2.8 to 8.3 hours for large molecules. It is generally well known that times of about 3t_{diffusion} to 5t_{diffusion} are required to obtain changes which are 95 to 99% complete for diffusion controlled processes. Thus, the times corresponding to 3t_{diffusion} for a 95% complete change are about 50 minutes to about 2 1/2 hours for small molecules, and about 8 1/2 hours to about 1 day for large molecules, and still longer for a 99% complete change, so that it is generally impossible to provide rapid, completed changes of chemical concentrations at the site of cells contained in conventional gel slabs, Petri dishes or large gel particles by the general means of changing external chemical concentrations (see, for example, Nilsson et al., Nature, 302: 629 - 630, 1983; Nilsson et al., Eur. J. Appl. Microbiol. Biotechnol., 17: 319 - 326, 1983).

The optical path length associated with optical measurements on cells in conventional gel slabs or Petri dishes can often be equal to a significant fraction of the thickness, or the entire thickness, of the gel slabs or petri dish material. For this reason, the size of conventional, macroscopic gel preparations also places limitations on optical measurement methods which can be used with the gel preparations, as light absorbtion, light scattering and auto fluorescence can be significant, and result in difficulty in making accurate measurements on individual or small numbers of cells within such macroscopic gels.

A major disadvantage of viable plate enumerations is slowness: typical determinations require one-half to several days, and are also labor- and materials-intensive. Another major disadvantage is the relatively inability to readily change the chemical or physical conditions present during incubation, as the macroscopic size of petri dishes results in slow diffusion times for changes of chemical conditions, slow temperature response times, and a general inability to position the cells on the surface of a petri dish in close proximety of conditions, or sources of influence, which can alther cell survival, cell growth or other cell behavior.

In view of these limitations of prior methods for determining growth of biological entities, particularly cells, significantly more rapid and automated methods for biological growth determination which allow rapid changes in growth conditions to be made, which are based on a small number of biological entities, preferably individual biological entities, and therefore do not require prior incubation, and which are capable of determining biological growth under a variety of conditions, using samples which contain small numbers of biological entities, would be highly desirable.

Many assays and tests are based on exposure of cells to compounds and agents followed by an incubation period during and/or after which the amount of biological entity growth is determined, so that the biological entity killing effects, biological entity inhibitory effects and biological entity growth stimulating effects, and the like, can be directly determined. Such assays and tests are widely used, and have important applications in fields such as clincial microbiology, cancer diagnosis and treatment, environmental science, food safety, toxicology, and research and development in basic and applied biology.

Prior methods for determining the effect of compounds and agents on the growth of biological entities generally have the disadvantage of either not providing a direct test of the effects of the compounds and agents, but rather infer the effects, or the direct tests are slow, and often require significant manual steps with the attendent attributes of cost and human error.

The prior methods for determining antimicrobial susceptibility of microorganisms of clinical significance provides an illustrative example. Generally, two broadly different types of assays and tests are presently employed. First, the traditional methods involve collection of a clinical sample such as urine, blood, fecal matter, CNS fluid, or the like, wherein such sample material is either directly spread onto petri dishes or the sample material is used to inoculate one or more containers with enrichment media in order to grow up the relatively small numbers of microbial cells which may be present. In one approach, several petri dishes containing various compounds and agents at different concentrations can be used directly with the primary or original sample. Alternatively, culture of the primary sample directly on petri dishes can be used, giving a viable count, and, often in the prior art, the colonies of 10⁷ to 10⁹ cells can be used as inoculum for subsequent tests. In the general case wherein the subsequent tests are based on growth, an additional series of petri dishes can be used, each with different compounds and agents present at different concentrations, or the colonies from the initial petri dish culture can be used to inoculate bulk media.

Growth in bulk media, to which various agents and compounds have been added, can then be carried out. Important general examples involve liquid suspension culture, with measurement of parameters such as light scattering, electrical impedance changes, or carbon dioxide production. By comparison to control suspensions to which the cells, but not the various agents and compounds, have been added, the effects of such agents and compounds on average cell growth can be determined.

Other bulk media tests are carried out in gel medium, such as the (Kirby-Bauer) disk diffusion tests for antimicrobial susceptibility, wherein a gel containing growth medium has a large number of cells placed on the surface, thereby inoculating the gel surface (see, for example, Finegold and Martin *Bailey and* *Scott's Diagnostic Microbiolgy*, C. V. Mosby, St. Louis, 1982). One or more discs containing a relatively high concentration of candidate antimicrobials are placed into or on the gel, such that outward diffusion of the candidate antimicrobial results in a concentration gradient of candidate antimicrobial concentration. Following incubation, the size and characteristics of the regions which have inhibited or killed cells is observed, and used as the basis for determining the effect of the candidate antimicrobial compounds on cell growth.

General properties of such bulk medium growth assays and tests is that the test result is based on the average response of the cells, prior culture of primary sample cells is typically required to obtain colonies for inoculation of the bulk medium, with the result that the overall assay or test procedure is time consuming, often requiring one, two or more days, and the bulk assays and tests do not reveal any cell population heterogeneity of antimicrobial susceptibility.

Significantly more rapid and automated methods for assaying and testing the effects of agents and compounds on cell growth is therefore desireable. Determinations based on a small number of cells, preferably individual cells, and which therefore do not require prior incubation and growth for determination of cell growth in samples which contain small numbers of cells, would be highly desirable.

The viable cell enumeration, or count, is the number of viable cells per volume of sample, and is denoted here by ρₛ. The determination of a viable enumeration is important to and widely used in biological research, clincial microbiology, cancer diagnosis and treatment, environmental science, food safety, toxicology, and research and development in basic and applied biology. Present methods for viable enumeration are slow and generally labor intensive, and many newer methods which purport to give an enumeration are not based on actual viable cell counts. Instead, many of these methods measure some average property of a large number of cells which, under well defined conditions, correlates with a count, but which under other conditions generally does not correlate accurately with a viable count.

Thus, methods which provide more rapid viable enumeration of biological entities than conventional viable plating, which is based on viability determined directly by biological growth of biological entites, or determined less directly by vital stains, which is capable of measuring large numbers of biological entities, which is capable of more accurate quantitation, and which is capable of more automation, would be highly desirable.

The determination of properties of a mixed biological problem is frequently of interest because biological systems seldom are comprised of one type of biological entity, and yet it is often desirable to measure the amount or behavior of, one or more types of biological entities of the mixed population. Thus, it is common to have biological system samples which contain more than one type of small multicellular organisms, groups of cells, individual cells, protoplasts, vesicles, spores, organelles, parasites, viruses, nucleic acid molecules, antibody molecules, antigen molecules, and aggregates of molecules, whereas the determination of one or more of the different types is of specific interest.

It is useful to illustrate prior approaches to this general problem by considering prior methods for determining the properties of mixed cell populations, as mixed cell population determinations adequately present the general problems. Determination of cell properties include determinations based constituative properties such as identification relating to speciation, classification by presence of specific antigens or specific genes, and determinations based on behavior properties. Such properties include growth as reflected by the lag time and the doubling time, viable enumeration or number of viable cells per volume, effects of compounds and agents on growth, such as antimicrobial susceptibility in terms of minimum inhibitory concentration or minimum bactericidal concentration, or such as chemotherepeutic agent susceptibility. These determinations are generally important to and widely used in clincial microbiology, cancer diagnosis and treatment, environmental science, food safety, toxicology, and research and development in basic and applied biology. However, essentially all determinations of growth, viable enumeration and effects of compounds and agents on growth must presently be carried out using monopopulations, that is samples or preparations of cells which are all of the same type.

Although many monopopulation methods can be used with some success to test majority cells in mixed populations which have a small fraction of cells different from the majority, such methods fail if the minority cells are significant in number, biological activity or growth compared to the majority cells. The general inaplicability of previous methods to mixed populations results from the fact that most methods, other than conventional viable plating, are based on the effects or behavior of the all the cells in the sample, and thereby the average behavior of the cells in the sample. In general, therefore, a mixed population contains two or more cell types, present *a priori* in unknown numbers, such that the result of a determination is based on an unknown average.

For example, consider a mixed cell population consisting of two cell types, one (A) fast growing, and the other (B) slow growing, in the case that the sample contains 1% of A and 99% of B. If it were desired to determine the growth rate by by counting the total number of cells as a function of time, and the sample were incubated, the relative composition of the population would continuously change, such that at after some time both A and B types would be present in approximately equal numbers, and later type A would predominate. During such a process the population average would initially be representative of type B, after some time would be representative of the average behavior of types A and B, and later would be representative of type A. Without *a priori* knowledge of the initial numbers of each cell type, and the different growth rates of A and B under the conditions of the incubation, it is impossible to determine the underlying behavior of cell types A and B, as in general it would not be known that only two cell types were present.

To continue this example, if instead of total cell number an activity such as metabolic product rate (e.g. CO₂ production) or metabolic effect (e.g. electrical impedance rate of change) were measured, even less insight would be available, because only the rate of change of a total parameter is measured, without any knowledge as to the number and types of cells which together produced the overall activity. This indicates a fundamental difficulty of all total population methods: they cannot determine growth or activity based on measurements of a large number of cells unless all the cells are essentially the same, or those cells that are different represent a small fraction of the total population. Thus, total population activity methods are generally inapplicable to mixed biological populations.

Such total population methods include optical techniques such as those which measure the change in light scattering due to many cells in a liquid suspended culture (See, for example, Edberg and Berger, in *Rapid Methods and* *Automation in Microbiology and Immunology*, K. O. Habermehl, Ed., Springer-Verlag, Berlin, 215 - 221, 1985), and a variety of metabolic activity based techniques which measure changes due to many cells in an analyzed sample, of which examples are changes in extracellular pH (See, for example, *Cowan and Steel's* *Manual for the Identification of Medical Bacteria*, Cambridge University Press, Cambridge, 1974; *Manual of Methods for General Bacteriology,* P. Gerhardt, (Ed), American Society for Microbiology, Washington, 1981), carbon dioxide release (see, for example, Courcol et al., J. Clin. Microbiol., 25: 26 - 29, 1986; Manca et al., J. Clin. Microbiol., 23: 401 - 403, 1986), electrical impedance (see, for example, Stewart, J. Exp. Med., 4: 235 - 245, 1899; Eden and Eden *Impedance Microbiology*, Research Studies Press, Letchworth, 1984; Hadley and Yajko, in Instrumental Methods for Rapid Microbiological Analysis, Nelson (Ed.), VCH, Weinheim, 193 - 209, 1985; Bishop and White, J. Food Protect., 49: 739 - 753, 1986), chemiluminescence (see, for example, Neufeld et al., in *Instrumental* *Methods for Rapid Microbiological Analysis*, Nelson (Ed.), VCH, Weinheim, 51 - 65, 1985; *Rapid Methods and Automation in Microbiology and Immunology,* Habermehl (Ed.), Springer-Verlag, Berlin, 1985) or fluorescence (see, for example, Rossi and Warner in *Instrumental Methods for Rapid Microbiological* *Analysis*, Nelson (Ed.), VCH, Weinheim, 1 - 50, 1985; *Rapid Methods and Auto**mation in Microbiology and Immunology*, Habermehl (Ed.), Springer-Verlag, Berlin, 1985). A disadvantage of all such metabolic activity methods is that they are based on combined effects of a large number of cells, and therefore generally require an initial process, based on plating, to obtain initial colonies for purposes of inoculation of the analyzed sample, such that the determinations based on many cells at least are based on a monopopulation, i.e. a population comprised nominally of the same type of cells. For this reason, although a total population cell determination may itself be rapid, it is generally preceeded by a viable plating method, or its equivalent, or order to obtain a monopopulation, and this process is slow. Thus, the total analysis time, counted from receipt of a primary or non-plated mixed population sample to a desired determination, is the sum of both, and therefore still long.

Certain constituent properties of mixed populations can be directly determined using methods applied directly to a mixed population, but only in the sense that the assayed constituent is determined to be present, and without knowledge that some cells in the sample might not have the constituent. For example, specific ligand binding assays such as immunoassays or genetic probe assays can be used directly with a mixed population to determine, and if needed to quantitate, the presence of specific, pre-specified antigens or nucleic acid sequences (see, for example, *Alternative immunoassays*, Collins (Ed.), Wiley, 1985; Mullis et al U.S. Patent No. 4,683,195). However, such determinations do not reveal anything about other cells which may be in the sample which do not have the constituent, nor do they determine the number of cells present which have the assayed constituent material.

Further, certain important tests, such as antimicrobial susceptibility, generally involve quantitative determination of the concentration at which an antimicrobial is effective, such that the MIC (minimum inhibitory concentration) and the MBC (minimum bactericidal concentration) is often determined (see, for example, Fine-gold and Martin *Bailey and Scott's Diagnostic Microbiolgy*, C. V. Mosby, St. Louis, 1982). Such quantitative results can be obtained directly only through testing the response of cells to candidate antimicrobial compounds at different concentrations, and cannot be actually determined directly from detection or measurement of the total amount of specific antigen or specific nucleic acid sequence which is present in a sample. Instead, present practice is to infer useful, or likely to be useful, concentrations of antimicrobials based on general experience, and not on the basis of a specific constituent test carried out on a sample.

Generally, therefore, although it is possible to determine the presence, and the total amount, of particular cell constituents, it is not possible to make such determinations based on cell behavior, such as activity or growth, directly on a mixed population, which is a sample or preparation consisting of two or more different cell types. This general limitation requires that present methods utilize some prior step to obtain cells which are all of the same type, including methods such as dilution and plating to obtain colonies of the different cells which can then be used to inoculate and grow monopopulations, or alternatively growth on selective growth media which favors growth of certain types of cells over others, thereby also resulting in obtaining a monopopulation. These present methods have the disadvantage that this prior step, resulting in a monopopulation, is generally slow. For example, dilution of a primary sample followed by plating on a petri dish to obtain colonies for subsequent preparation of monopopulations requires spreading of a suitably dilute suspension of cells on the surface of a petri dish, followed by growth of initial individual cells (or colony forming units, CFU) until visible colonies are obtained.

Similarly, preparation of a monopopulation by using specific inhibitors or cytotoxins generally requires high toxic specificity, so that only one type of cells survives exposure to the inhibitors or killing agents, and that the surviving cells are not significantly stressed, damaged or temporarily altered in a way that effects their subsequent use. As a result, cells obtained by exposure to a selective toxic medium generally require a period of recovery on non-selective medium prior to determination of the monopopulation cell properties.

Growth of cells on selective media is another widely used method for obtaining cells of one class or type. If specific useful properties of one type of cells is known, for example, resistance to a particular antimicrobial compound, then a mixed population can be placed in a growth medium with that antimicrobial compound, and a total population measurement attempted. However, even if some cells do not grow, they may be only inhibited by the antimicrobial, or if killed by the antimicrobial, may still retain certain enzyme and metabolic activities for a period of time. In general, then, an incubation period must be used, during which non-resistant cells are killed, or are made to represent a smaller and smaller fraction of the mixed population as the resistant cells grow. For these reasons, if the non-resistant cells comprise a significant fraction of the sample cells, then a significant growth period must be used in order to be reasonably sure that a total population measurement is based predominantly on the resistant cells. This process of growth using a selective medium is essentially equivalent however, to obtaining a monopopulation, and is therefore not a method which can be directly and rapidly applied to a mixed population sample.

Overall, present methods for making determinations on mixed populations are generally slow and often labor intensive, because of the need to first obtain a mono populations or pure populations. This need for a prior mono population is fundamental, as present rapid methods based on activity or growth are total population methods, or determine specific constitutent cell material without determining how many, or what types, of cells are present.

The only established method capable of dealing directly with mixed populations is conventional plating following a suitable dilution, such that the cell suspension spread over a petri dish surface has a high probability of having well separated cells. these cells are subsequently grown up into visible colonies of 10⁷ to 10⁹ cells by incubation for a time corresponding to 22 to 30 generations. (See Sharpe, in *Mechanizing Microbiology*, A. N. Sharpe and D. S. Clark (Eds.) Charles C. Thomas, Springfield, 19 - 40, 1978). Even for rapidly growing cells, such as bacteria with a doubling time under optimal conditions of 20 to 30 minutes, the required incubation time is long, about 7 to 15 hours, and for many other microorganisms and mammalian cells the required incubation is several days.

It is recognized that conventional viable plate enumeration has great importance, and is the standard method to which all others are compared. Viable plating typically involves the spreading of a suspension of cells onto the surface of a gel-containing petri dish. Viable plating can be generally used with mixed populations, because the process of diluting and spreading the sample onto a petri dish surface serves to (statistically) separate individual cells, and is the only present process, other than specific constituent assays, which is capable of directly using mixed population samples, but such viable plating typically requires about 1/2 day for biological entities such as very rapidly growing cells, and for other, more slowly growing biological entities often requires several days.

It is, therefore, highly desireable to have a much more rapid method for making determinations using a process which can be used directly and rapidly with a mixed population sample, does not require a prior incubation, which does not require restrictive assumptions or highly specific pre-specification about the genetic or immunologic-reacting composition of the biological entities, and which also can utilize a relatively small number of sample biological entities.

Prior methods for measuring microdroplets (MDs) surrounded by non-aqueous fluids also suffer from significant disadvantages, and have not demonstrated an ability to flexibly manipulate such MDs physically. For example, prior use of liquid microdroplets by Rotman to investigate the activity of individual molecules of β-D-galactisidase involved the formation of such liquid microdroplets (LMDs) by spraying. In this process air-borne LMDs were formed from a solution, impacted upon a stationary silicone fluid on a solid surface, and then settled, such that the resulting LMDs were not further manipulated, but instead manually observed using fluorescence microscopy (PNAS, 47: 1981 - 1991, 1961).

Similar use of LMDs was used to measure the activity of intracellular β-D-galactosidase of bacteria contained within LMDs, and involved manual observation of stationary LMDs surrounded by a silicone fluid using fluorescence microscopy (Revel et al, PNAS 47: 1956 - 1967, 1961).

Likewise, prior methods involving measurement of GMDs surrounded by a non-aqueous fluid has involved the detection of microorganisms in GMDs surrounded by stationary mineral oil, with light absorbance or fluorescence changes manually observed by microscopy. Further, although it has been suggested that GMDs can be measured by the use of flow cytometry wherein non-aqueous fluids replace the established aqueous fluidics, such measurement involves only the passage of GMDs suspended in a non-aqueous fluid through a flow cytometery (Weaver, Biotech. and Bioengr. Symp. 17: 185 - 195, 1986) and does not result in the purposeful manipulation of GMDs within the non-aqueous fluid.

For these reasons, it would be highly desirable to have improved means for physically manipulating microdroplets surrounded by non-aqueous fluids.

In addition, prior use of microdroplets (MDs), although indicating that information relating to the viable enumeration can be estimated, is highly approximate, generally utilizes only one or a small number of MD sizes, is dependent upon interpretation of visual images of MDs while observing MDs by microscopy, and suffers from being tedious and difficult. For example, the investigation by Rotman of the activity of individual moleucles of the enzyme β-D-galactosidase depended upon the visual inspection of a relatively small number of MDs by fluorescence microcospy (PNAS, 47: 1981 - 1991, 1961). Further, although this investigation of individual enzyme activity is important and fundamental, the methodology is sufficiently tedious and difficult that this use of MDs has not been repeated or extended, and has not been available in a more automated or more quantitatively accurate version. A related investigation of bacterial β-D-Galactosidase activity utilized bacteria contained within liquid microdroplets (Revel et al, PNAS 47: 1956 - 1967, 1961).

Likewise, prior use of gel microdroplets (GMDs) has involved the detection of microorganisms in GMDs surrounded by mineral oil, with extracellular light absorbance or fluorescence changes manually observed by microscopy, with manual estimates of GMD diameter by comparision to a haemocytometer grid dimension, such that a very approximate estimate of the GMD volumes within one or a small number of GMD size ranges could be made (Weaver et al., Ann. N.Y. Acad. Sci., 434: 363 - 372, 1984; Williams et al, Ann. N. Y. Acad. Sci. 501: 350 - 353, 1987). Because of the tedious, manual nature of such determinations, typically the diameters of 100 or fewer GMDs were estimated, and used to approximately estimate the consistency of the observed number of occupied GMDs with Poisson statistics.

Although approximate consistancy with Poisson statistical formulae was found in prior use of liquid and gel microdroplets, such prior use of MDs suffered from inaccuracy of MD size measurement, an inability to readily measure large numbers of MDs, and a resultant inability to apply mathematical statistical analysis in a more thorough, iterative fashion that would provide self-consistent mathematical determinations of the occupation of the MDs, and, thereby, an enumeration. Further, such prior use of MDs did not provide a stringent basis for determining the viability of any biological entities contained within MDs, and therefore did not provide the type of measurent yielded by conventional viable plating methods.

Further, prior use of liquid microdroplets (LMDs) has been limited to demonstrating that the occupation of LMDs by single enzyme molecules (Rotman, PNAS 47: 1981 - 1991, 1961) or by bacteria (Revel et al, PNAS 47: 1956 - 1967, 1961), and of GMDs by bacteria and yeast, is consistent with Poisson statistics (Weaver et al., Ann. N.Y. Acad. Sci., 434: 363 - 372, 1984; Williams et al, Ann. N. Y. Acad. Sci. 501: 350 - 353, 1987). However, such prior demonstrations do not have high accuracy, as the measurements of such LMDs and GMDs surrounded by a non-aqueous fluid are based on visual observation of a relatively small number of MDs using light microscopy or fluorescence microscopy, and do not involve a large number of measurements of individual MD diameters, from which MD volume for each adherent, somewhat deformed spherical MD can be estimated within each of several ranges of MD volumes. For this reason, instead of being directed towards a determination of an enumeration, these prior demonstrations emphasized approximate consistancy with Poisson statistics. Futher, these prior uses of MDs have not involved determination of the viability of cells based on growth. For these reasons, prior use of MDs has not been directed towards a rapid determination of a viable enumeration.

Gel microdroplets (GMDs) provide a general means for improving significantly both cell analysis methods and cell isolation methods. In order to utilize GMDs more effectively, it is highly desireable to have improved means for detecting, measuring and characterizing individual GMDs independent of the cell content of a GMD. Examples of are detection of GMDs for signal triggering purposes, distinction of GMD-entrapped and non-entrapped (free) cells, measurement of GMD volume (V_{GMD}), and measurement of number of co-entrapped or co-provided binding sites.

Prior methods for measuring GMDs involve optical measurements on GMDs made from unlabeled gel, and which utilize dyes which are dissolved in the aqueous liquid of the gel (see U.S. Patents No. 4,401,755 and 4,643,968 by Weaver, the teachings of which are incorporated herein by reference). For example, visible light microscopy has been used to observe larger microorganisms such as yeast within agarose GMDs, and in such cases it is found that the agarose gel itself is faintly seen because pure agarose scatters light weakly and also has no significant light absorption which would impart a color to the gel. Many other gel materials, such as alginate scatter more light and are thereby more optically distinguishable, but also have no significant color. In the prior GMD measurements, GMDs made from pure agarose gel were suspended in mineral oil, in order to retain charged extracellular metabolites within the small volume of the GMDs, and an additional attribute of such GMD preparations is that the difference in optical index of refraction renders the pure agarose GMDs visible by light microscopy.

Measurement relating to detection of active microorganisms in GMDs has been made by utilizing a light absorbing or colorimetric dyes dissolved in the aqueous medium of the agarose gel of the GMDs. An exemplary case is the use of bromothymol blue, a pH sensitive light absorbance or colorimetric indicator dye which changes color from "Blue" at an initial pH of about 7.6 to "Yellow" at a significantly lower pH, is provided by dissolving the dye in the medium in which the cells are suspended. GMDs are then created by dispersion in mineral oil, such that the cells are entrapped in the GMDs, but the solubilized indicator dye is retained within GMDs only because of the dye's relatively poor solubility in the mineral oil which surrounds the GMDs (see Weaver et al., Ann. N.Y. Acad. Sci., 434: 363 - 372, 1984; Weaver, Biotech. and Bioengr. Symp. 17, 185 - 195, 1986; Williams et al., Ann. N. Y. Acad. Sci., 501: 350 - 353, 1987).

In such cases of solubilized dyes, it is sometimes observed that the GMD boundary and the aqueous liquid volume associated the GMD are not the same, because some gel shrinkage can occur, with the result that a thin layer of aqueous liquid medium surrounds each GMD. Although the existence of a non-contained aqueous volume is not detrimental in some applications, e.g. incubation and measurment of GMDs resting on a solid surface, in other applications or manipulations the non-contained aqueous volume results in difficulties. For example, during stirring of GMDs suspended in mineral oil, collisions of GMDs with walls of a macroscopic container can result in partial loss or chemical contamination of the aqueous medium within the colliding GMDs. Likewise, collisions can occur between GMDs, such that some aqueous liquid medium can be exchanged between GMDs, which also results in partial transfer of the solubilized dye. In the illustrative case of pH indicator dyes used to measure microbial metabolic activity within GMDs, it is found that the indicator dye can be a significant buffer for pH change. This has the consequence that metabolic acid can be exchanged between GMDs by such collisions, not only by transfer of solubilized H⁺, but also by transfer of "bound" H⁺ related to the transfered pH indicator dye.

Similarly, while the use of one or more solubilized fluorescent indicator dyes allows more sensitive and rapid measurement of microbial activity in GMDs suspended in mineral oil than does the use of colorimetric indicator dyes, the problems associated with the use of solubilized dyes are the same. Thus, many prior measurements of GMD properties have small optical signals because most gel materials scatter light poorly, have weak light absorbance or color, or have autofluorescence. Further, solubilized dyes are not always adequately confined within GMDs if the dye is dissolved in the the aqueous liquid within the gel, and the GMDs are surrounded by mineral oil. For these reasons it would be highly desirable to have GMDs with improved measurablility, and processes for measuring such GMDs.

Previous studies of gel microdroplets have demonstrated that the suspension of gel microdroplets (GMDs) in a non-aqueous fluid such as mineral oil results in retention of microbial metabolites such as metabolic acids. This in turn allows detection and enumeration of acid-producing microorganisms through the use of colorimetric and fluorescent pH indicators (Weaver et al., Ann. N.Y. Acad. Sci., 434: 363 - 372, 1984; Williams et al, Ann. N. Y. Acad. Sci. 501: 350 - 353, 1987). In the previous work, observation of pH changes by optical means such as colorimetry or fluorescence was accomplished using fluorescence microscopy. However, such prior use demonstrated that insignificant chemical communication of highly water soluble compounds, such as metabolic acids, occurs between GMDs even when they are almost contacting. Thus, such GMDs are found to be essentially chemically isolated, and cannot readily have the chemical composition of the aqueous interior of such GMDs altered or modified.

An attribute of these previous GMD methods is that GMDs surrounded by mineral oil (non-aqueous surrounded GMDs) were provide a chemical isolation of GMDs, such that many chemicals are retained within GMDs. This retention within the very small volume of a GMD is the basis for an important class of measurements on individual cells, individual enzyme molecules, microcolonies, and the like. In this previous use of GMDs, chemical retention is achieved by surrounding GMDs by a non-aqueous fluid which has solubility properties which essentially exclude the dissolution of many chemicals, particularly charged or ionic species such as acids. For this reason, such chemicals do not significantly partition into the non-aqueous fluid, and cannot therefore be transported through the non-aqueous fluid by a combination of diffusion and convection.

Although this is highly desirable in order to retain the extracellular products of biologically active entities within a GMD, and also to provide or retain the chemical assay reactants used within a GMD as the basis of an extracellular and/or intracellular assay, the previous use has resulted in an inability to alter the chemical composition, for chemicals insoluble in the non-aqueous fluid, of the GMD interior once the GMDs were created and surrounded by a non-aqueous fluid.

It would, however, be highly desirable to be able to alter the chemical composition of GMD interior fluids at one or more times after the GMDs have been surrounded by a non-aqueous fluid, under controlled conditions, and without the necessity of removing the GMDs from the non-aqueous fluid followed by a re-suspension or re-surrounding of the GMDs by a non-aqueous fluid.

### Summary of the Invention

The present invention pertains to a method of micro-manipulation comprising the steps of:
a) forming an aqueous microdroplet which contains the subject of the micromanipulation and a force coupling component having ferromagnetic, diamagnetic, paramagnetic, dielectric, electrical charge, electrophoretic and/or optical pressure properties,
b) surrounding the microdroplet with a non-aqueous medium having ferromagnetic, diamagnetic, paramagnetic, dielectric, electrical charge, electrophoretic, and/or optical pressure properties which differ from those of the force coupling component, and
c) applying a physical force selected from electrical, magnetic, acoustic and optical forces whereby a differential response to the applied force of the force coupling component relative to the non-aqueous medium causes micro-manipulation of the subject of the micromanipulation, the subject of the micromanipulation comprising a cell, vesicle, spore, organelle, virus, nucleic acid molecule, antibody molecule or antigen molecule.

### Brief Description of the Figures

### Figure 1

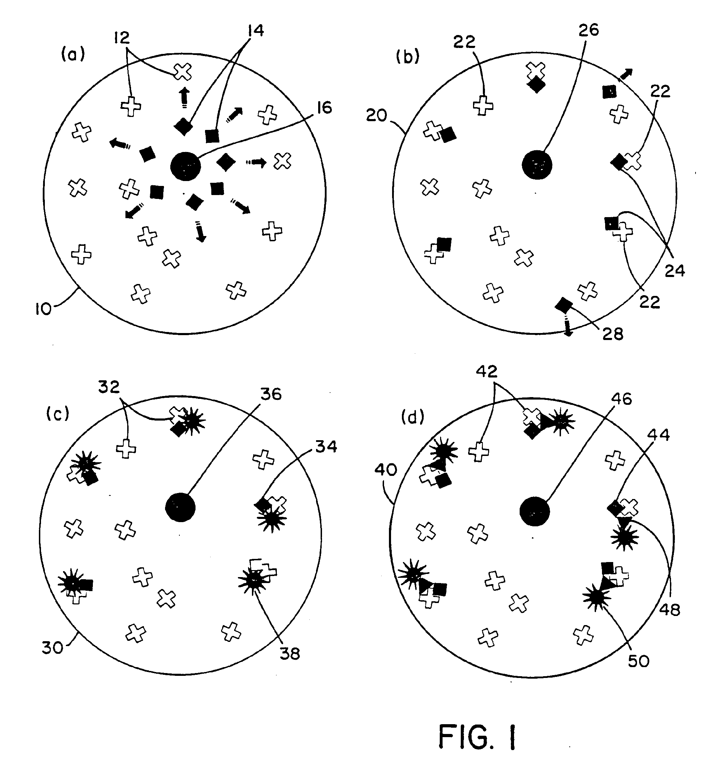

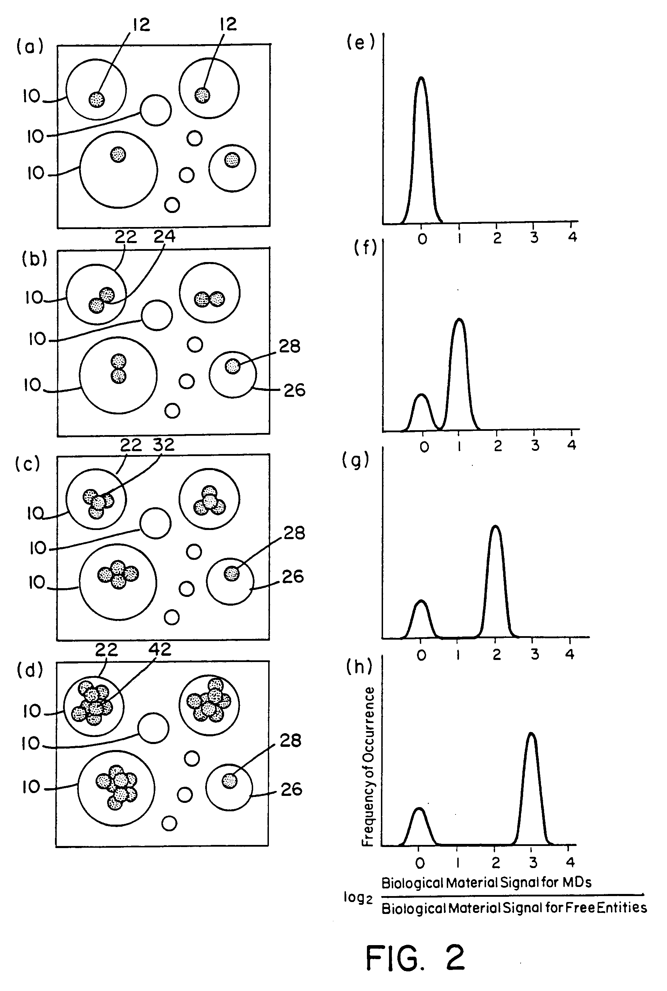

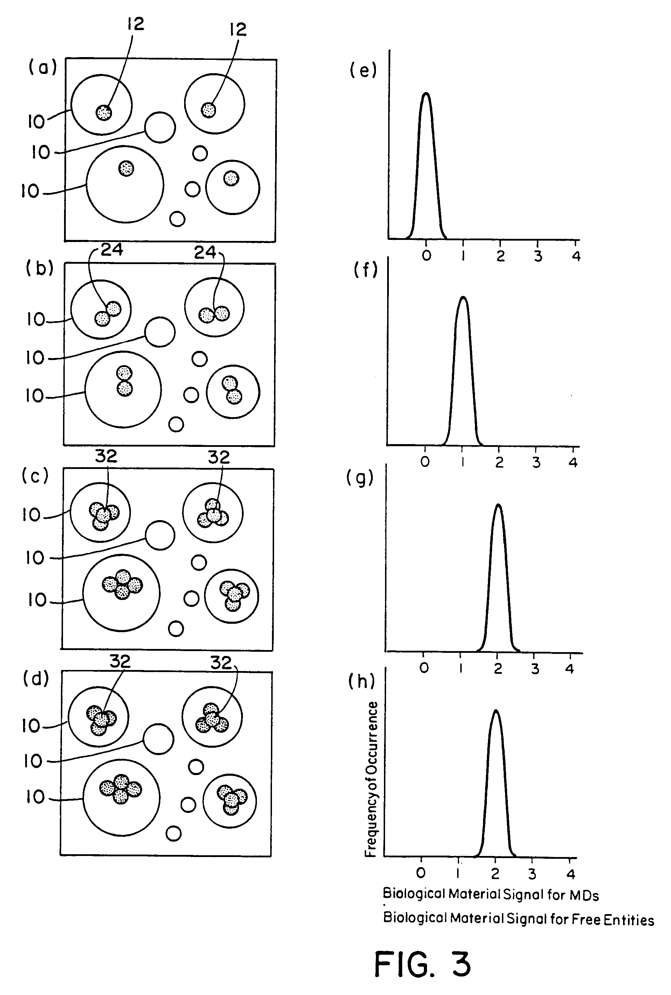

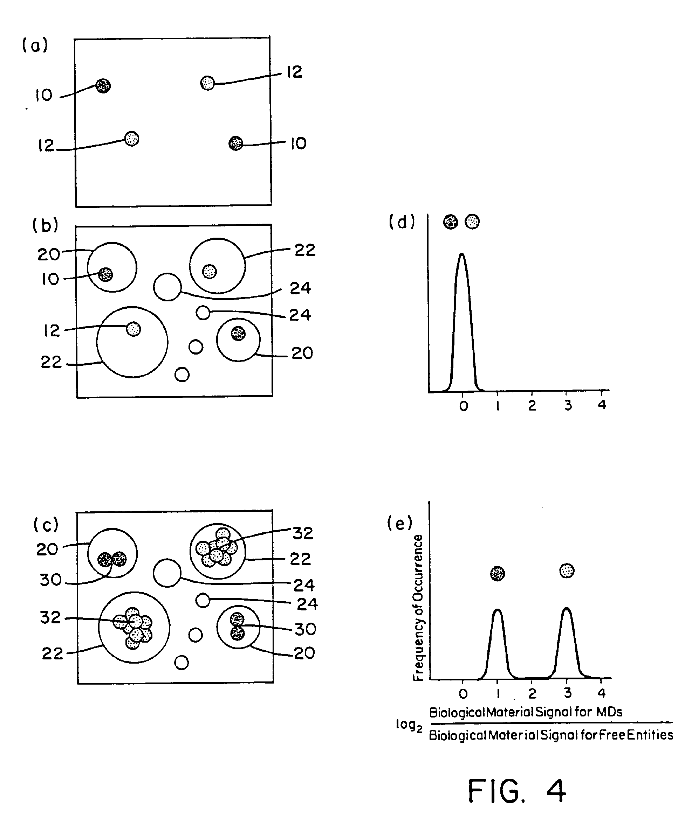

Part (a) of the figure illustrates a GMD (10) with provided binding sites (12) capable of capturing released molecules (14) originating from a biological entity (16) contained within the GMD. Part (b) shows a GMD (20) with some binding sites (22) having captured released molecules (24) originating from a biological entitiy (26) with other released molecules (28) leaving the GMD. Part (c) shows a GMD (30) with some binding sites (32) having captured released molecules (34) originating from a biological entitiy (36), and in turn binding labeling molecules (38). Part (d) shows a GMD (40) with some binding sites (42) having captured released molecules (44) originating from a biological entitiy (46), which have bound intervening molecules (48), and in turn binding labeling molecules (50).

### Figure 2

Part (a) of the figure illustrates GMDs (10) of several sizes prior to incubation, with four of the larger GMDs each containing an individual cell (12). Part (b) of the figure illustrates the same GMDs (10) after an incubation corresponding to one doubling time, with three of the larger GMDs (22) each containing a two-cell microcolony (24), and one larger GMD (26) containg a non-growing cell (28). Part (c) of the figure illustrates the same GMDs (10) after an incubation corresponding to two doubling times, with three of the larger GMDs (22) each containing a four-cell microcolony (32), and one larger GMD (26) containg a non-growing cell (28). Part (d) of the figure illustrates the same GMDs (10) after an incubation corresponding to three doubling times, with three of the larger GMDs (22) each containing a eight-cell microcolony (42), and one larger GMD (26) containg a non-growing cell (28). Parts (e), (f), (g) and (h) are histograms of a signal proportional to the biological material based on the measurements of (a), (b), (c) and (d) respectively.

### Figure 3

Part (a) of the figure illustrates GMDs (10) of several sizes prior to incubation, with four of the larger GMDs each containing an individual cell (12). Part (b) of the figure illustrates the same GMDs (10) after an incubation corresponding to one doubling time, with four of the larger GMDs (12) each containing a two-cell microcolony (24). Part (c) of the figure illustrates the same GMDs (10) after an incubation corresponding to two doubling times, with four of the larger GMDs (12) each containing a four-cell microcolony (32). Part (d) of the figure illustrates the same GMDs (10) continuing the incubation but in the presence of a growth inhibitor, with three of the larger GMDs (12) each containing a four-cell microcolony (32). Parts (e), (f), (g) and (h) are histograms of a signal proportional to the biological material based on the measurements of (a), (b), (c) and (d) respectively.

### Figure 4

Part (a) illustrates a mixed population comprised of two types of cells, two cells of type A (10) and two cells of type B (12). Part (b) illustrates formation of microdroplets such that two microdroplets (20) each contain one initial type A cell (10), two other microdroplets (22) each contain one initial type B cell (12), and four other microdroplets (24) contain zero initial cells. Part (c) illustrates the same microdroplets as in part (b) after an incubation, showing two microdroplets (20) containing a two-cell microcolony (30) of A cells, two microdroplets (22) each containing an eight-cell microcolony (32) of type B cells (12), and four other microdroplets (24) contain zero cells. Part (d) illustrates a histogram for a signal responsive to biological material before an incubation, and Part (e) illustrates a histogram for a single responsive to biological material an incubation wherein cell type A grows more slowly than cell type B.

### Detailed Description of the Invention

### Microdroplets

This invention involves the use of microdroplets, which are very small volume entities comprised of liquid or gel material, and which can contain zero, one or multiple biological entities. More specifically, the term microdroplet (MD) includes both the gel microdroplet (GMD), the liquid microdroplet (LMD), with or without contained biological entities. Thus, unless restricted by specific use of the term "gel microdroplet" or "liquid microdroplet", the term "microdroplet" refers to both gel and liquid microdroplets.

Liquid microdroplets (LMDs) are very small volumes of predominantly liquid material, which can contain solutions, typically aqueous solutions with inorganic and/or organic chemical compounds, and which can additionally contain biological entities. LMDs have volumes which are defined by a boundary comprised of another liquid, such as a non-aqueous fluid, or by a permeability barrier such as a membrane, such that the membrane is capable of retaining biological entities of interest within a LMD, and also capable of passing other biological entities such as molecules.

Although LMDs can be of any shape, LMDs are often approximately spherical because of the tendency of interfacial forces associated with the boundaries of LMDs to round up the deformable LMDs. Other forces, for example hydrodynamic shear associated with stirring a LMD suspension, adhesion to a surface, or gravity, tend to cause departure from a spherical shape. Further, LMDs which contain or occupied by entities whose volume is a large fraction of the LMD volume can result in LMDs which are non-spherical. Thus, for example, a cell surrounded by a thin coating of an aqueous solution, which in turn is surrounded by a non-aqueous fluid, is a LMD. Similarly, a non-biological particle surrounded by a thin coating of an aqueous solution, which in turn is surrounded by a non-aqueous fluid, is also a LMD. If spherical, LMDs have diameters between about 0.2µ to about 1,000µ, preferably between about 5µ and about 500µ. Generally LMD volumes are between about 8 *x* 10⁻¹⁵ to about 1 *x* 10⁻³ *ml*, preferably between about 1 *x* 10⁻¹⁰ to about 1 *x* 10⁻⁴ *ml*.

Liquid microdroplets can be formed by a variety of methods, which are generally well known, and include methods based on breakup of a liquid jet, a spraying process, and by dispersion. For example, an aqueous liquid jet issuing into air can be forced to breakup into liquid microdroplets of nearly uniform volume (see, for example, Kachel and Menke in *Flow Cytometry and Sorting*, Melamed et al (Eds), Wiley, New York, pp. 41 - 59, 1979), or by spraying an aqueous liquid (see, for example, Rotman, PNAS 47: 1981 - 1991, 1961). Likewise, the use of dispersion to create emulsions consisting of a non-continuous aqueous phase of aqueous liquid microdroplets is well established (see, for example, Weaver et al., Ann. N.Y. Acad. Sci., 434: 363 - 372, 1984).

GMDs are very small volume entities which comprise at least one gel region, and which provide a mechanical matrix capable of entraping or surrounding, without necessarily contacting, biological entities such as small multicellular organisms, groups of cells, individual cells, protoplasts, vesicles, spores, organelles, parasites, viruses, nucleic acid molecules, antibody molecules, antigen molecules, and aggregates of molecules. GMDs can consisit entirely of gel, in which case containment of biological entities can occur by entrapment of the biological entities by the gel matrix. The general ability of gel matricies to entrap or immobilize biological entities is well known, having been established for a variety of macroscopic gel preparations such as Petri dishes, gel slabs and gel beads (see, for example, *Immobilized Cells and Organelles, Vols. I and II*, Mattiasson (Ed), CRC, Boca Raton, 1983).

Alternatively, GMDs can consist of a shell of gel matrix material which surrounds at least one aqueous liquid region, in which case containment of biological entites can occur by entrapment of the biological entities by the gel matrix material, or can occur by surrounding biological entities with a shell of gel matrix material, with or without contacting the biological entities.

Further, GMDs can consist of a plurality of regions comprised of gel material and liquid material. Representative configurations of GMDs with a plurality of gel regions include a first gel region entirely surrounded by a second gel region, wherein the second gel region can be comprised of a gel material different from the gel material of the first gel region. Alternatively, a second gel region can be comprise of essentially the same gel material as a first gel region, but the second gel can contain different entities such as entrapped beads and macro-molecules, or the second gel can have distinquishable molecules such as fluorescent molecules attached to a constituent of the second gel matrix.

Similarly, GMDs can contain liquid regions which are surrounded by at least one gel region. Representative GMDs with such liquid regions include GMDs which consist of a shell of gel material which surrounds at least one liquid region, such as an aqueous liquid core surrounded by gel. Such GMDs provide a general means for entrapping biological entities without necessarily contacting the biological entities with a gel matrix, as it is only necessary that the gel matrix be impermeable to the surrounded biological entities, and that the gel matrix be sufficiently mechanically strong that such GMDs remain intact during any desired physical manipulation process of GMDs.

Liquid regions and gel regions of GMDs which contain no biological entities are termed non-biological regions of a GMD.

Although GMDs can be of any shape, GMDs are often approximately spherical, with diameters between about 0.2µ to about 1,000µ, preferably between about 5µ and about 500µ. Generally GMD volumes are between about 8 *x* 10⁻¹⁵ to about 1 *x* 10⁻³ *ml*, preferably between about 1 *x* 10⁻¹⁰ to about 1 *x* 10⁻⁴ *ml*.

The term gel refers to a porous matrix with a high water content. Structures have the ability to entrap biological entities while allowing transport of many molecules within the aqueous medium of the gel matrix. The gel matrix can also contain a chemical solution, typically an aqueous solution with inorganic and/or organic chemical compounds. For example, the gel matrix can contain a physiologic solution or cell growth medium, which is comprised of inorganic ions and molecules and/or organic ions and molecules. Representative natural gel material for creation of GMDs includes kappa-carrageenan, iota-carrageenan, sodium alginate, furcelaran, zein, succinylated zein, succinlylated cellulose, agarose, collagan, fibrin, proteoglycans, elastin, hyaluronic acid and glycoproteins such as fibronectin and laminin, and other naturally occuring extracellular matricies, or the like. Representative synthetic gelable material synthetic water soluble polymers include those formed from vinyl pyrolidone, 2-methyl-5-vinyl pyrridine-methyl acrylate-methacrylic acid copolymer, vinyl alcohol, vinyl pyrridine, vinyl pyrridine-styrene copolymer or the like.

GMDs can be created by a variety of methods, including the subdivision of a macroscopic gel volume, but preferably GMDs are formed or created by converting an aqueous suspension into liquid microdroplets, followed by formation of a gel state from the liquid state of the liquid microdroplets. Liquid microdroplets (LMDs) are very small, deformable volumes of liquid which are surrounded by another distinct fluid, either liquid or gas, or are coated by a membrane material. A general process for creating GMDs involves first creating LMDs, wherein the LMDs are created from a liquid which contains gelable material, such that upon subsequent exposure to gelation conditions, the LMDs are transformed into GMDs. Formation of the gel state can be caused by a variety of well known gelation processes, including temperature changes, ion concentration changes, chemical concentrations, enzyme catalysis, and photopolymerization.

The associated gelation processes may be reversible without hysteresis, reversible with hysteresis or irreversible. In the case of reversible gelation without hysteresis, LMDs can be converted into GMDs, and GMDs can be converted into LMDs, by simply reversing the conditions, for example returning to a temperature which first caused gelation. In the case of reversible gelation with hysteresis, LMDs can be converted into GMDs, and GMDs can be converted into LMDs, by reversing the conditions beyond the conditions needed to cause gelation, for example returning to and then passing a temperature which first caused gelation. In the case of irreversible gelation, the conditions for reversing the gelation process cannot be achieved without creating conditions which are harmful to the biological entities contained in the LMDs or GMDs. One example of irreversible gelation is the formation of GMDs created by photopolymerization.

In one general procedure the liquid suspension is forced through a nozzle or vibrating orifice to form a liquid stream which breaks up, either because of the surface tension of a capillary jet, or by application of a shearing force, to form liquid microdroplets. Subsequently the liquid microdroplets are gelled by exposing the liquid microdroplets to conditions such as a temperature change, or by directing the liquid microdroplets into a solution containing ions which cause gelation. One attribute of the nozzle or vibrating orifice GMD creation method is that most GMDs are about the same size.

Another method for creating GMDs involves the first creation of a macroscopic gel volume, followed by subsequent fragmentation, cutting, disruption, or subdivision of the macroscopic gel volume such that a plurality of very small volume gel fragments or gel particles are created. This general method emphasizes that GMDs need not be spherical, nor even approximately spherical. Instead, it is only necessary that GMDs consist of very small volumes of gel material, with volumes between about 8 *x* 10⁻¹⁵ to about 1 *x* 10⁻³ *ml*, preferably between about 1 *x* 10⁻¹⁰ to about 1 *x* 10⁻⁴ *ml*.

It is generally preferred to use a dispersion method for creating GMDs from a liquid suspension, as dispersion methods are simpler, less expensive and generally freeer from clogging problems than are fluid jet methods. The dispersion methods consist of dispersing the liquid suspension into an immiscible liquid such as a heavy alcohol, mineral oil or silicone fluid, by means such as stirring or vortexing the liquid suspension and immiscible liquid together, thereby creating liquid microdroplets surrounded by the immiscible liquid. The liquid microdroplets are gelled during or after the dispersion process by any of a variety of well known gelation processes such as ion exchange or temperature change. Dispersion methods generally create GMDs with a relatively wide range of sizes, for example diameters of about 5µ to 500µ.

GMDs can also be formed by the process of fragmenting, cutting, disrupting or otherwise converting a macroscopic volume of gel into very small volume gel particles, such that said GMDs can have irregular shapes. For example, a macroscopic gel slab can be formed in which biological entities such as cells are entrapped at random positions, the gel slab can be cooled to a low temperature, and the gel slab then mechanically impacted so as to fragment the macroscopic gel into pieces, many of which have a very small volume and thereby constitute GMDs.

GMDs can also be formed by processes which cause a gel coating to form around one or more entities, such that the gel entirely surrounds, or essentially surrounds, the entities. For example, by contacting cold cells with a warmer solution of material which contains gel material which gels upon cooling, a coating of gel can be formed around the cells, such that the cells are thereby incorporated into GMDs. In this case the GMDs can be markedly non-spherical, as the gel coating often forms with the shape of the cells. Similarly, non-biological entities such as cell culture microcarrier beads, soil particles and food particles can be incorporated into GMDs by gel-coating processes, such that the resulting GMDs often have shapes which approximate the incorporated non-biological entities.

In those cases wherein GMDs are formed within a non-aqueous fluid, it is often desireable to transfer the GMDs into an aqueous medium, in order to expose biological entities to a variety of conditions relating to growth, metabolism, secretion, transmembrane potential development, membrane integrity and enzyme activity, and also to conditions which favor measurement and isolation of GMDs. An exemplary method for such transfer is gentle agitation if the GMD aqueous interiors contain suitable surfactant agents, including naturally occuring surfactants such as those present in serum.

### Composite Gel Microdroplets

Composite GMDs are GMDs which contain more than one distinguishable region of gel material or of liquid material, which gel or liquid materials are non-biological regions which may entrap or surround biological entities, and can be formed by several methods. Thus, a composite GMD is characterized by a plurality of non-biological regions which are further characterized by having at least one non-biological region having a first property surrounding substantially, or entirely, all of at least one non-biological region having a second property Composite GMDs can contain both gel and liquid regions, wherein liquid regions are surrounded by one or more gel regions, so that such composite GMDs must contain at least one gel region However, in order to be useful for making measurements on biological entities, and for isolating biological entities, at least some composite GMDs contain biological material

In one general method the aqueous suspending medium is provided with any cells, microbeads or other marker entities or force-coupling entities which are desired to be incorporated or entrapped in a first gel region. First GMDs are then formed from a first gellable material, using any of processes described elsewhere in this disclosure. The first GMDs are then suspended in a medium containing a second gellable material, which second gellable material may be of the same or different composition as the first gellable material. Additionally, the second gelable material can be comprised, partially or entirely, of material which has optical properties such as light scattering, light absorbance or colorimetric, fluorescence, time-delayed fluorescence, phosphorescence and chemiluminescence, so as to distinguish the second gel region from the first gel region. In this way, by using any combination of one or more of such methods, the second gel can provide composite GMDs, in this case of two distinguishable gel regions, termed GMD/GMDs, with desirable optical properties. For example, an optical signal for GMD diameter determination can be obtained from the second gel region while avoiding, with high probability, the contacting of first GMD entrapped cells with the second gel region.

More generally, it is useful to form composite GMDs wherein at least one region contains a first material with a first optical property selected from the group consisting of light scattering, light absorbance or colorimetric, fluorescence, time-delayed fluorescence, phosphorescence and chemiluminescence, and a second region contains a second, optically distinguishable material with optical properties selected from the group consisting of light scattering, light absorbance or colorimetric, fluorescence, time-delayed fluorescence, phosphorescence and chemiluminescence.

Alternatively, marker entities including beads, non-biological particles, crystals, non-aqueous fluid inclusions, viable cells, dead cells, inactive cells, virus, spores, protoplasts, vesicles, stains and dyes can be incorporated into the first gel region or first GMDs, and biological entities such as small multicellular organisms, groups of cells, individual cells, protoplasts, vesicles, spores, organelles, parasites, viruses, nucleic acid molecules, antibody molecules, antigen molecules, and aggregates of molecules can be incorporated into the second gel region in order to provide means for enhanced measurement of composite GMDs. In this case, composite GMDs with at least one gel region containing marker entities selected from the group consisting of beads, non-biological particles, crystals, non-aqueous fluid inclusions, viable cells, dead cells, inactive cells, virus, spores, protoplasts, vesicles, stains and dyes are provided Such composite GMDs are particularly useful in the case that the optical properties of the marker entities are selected from the group consisting of light scattering, light absorbance or colorimetric, fluorescence, time-delayed fluorescence, phosphorescence and chemiluminescence. Composite GMDs can also be characterized by a plurality of non-biological regions which are further characterized by having at least one non-biological region having marker entities surrounding substantially, or entirely, all of at least one non-biological region having a second property.

In order to form composite GMDs, several general processes can be used. One method for producing GMDs having a plurality of non-biological regions, with at least one non-biological region having different properties than at least one other non-biological region, consists of the following general steps: (a) forming GMDs of a first gel using any of the processes described elsewhere in this disclosure, (b) suspending the gel microdroplets in a material capable of forming a second gel, and (c) incorporating gel microdroplets of the first gel into gel microdroplets of the second gel, thereby forming gel microdroplets with distinct non-biological gel regions.

Another general method for producing GMDs having a plurality of non-biological regions, wherein at least one non-biological region has different properties than at least one other non-biological region, consists of the following general steps: (a) forming GMDs, (b) suspending said GMDs in a material capable of forming LMDs, and (c) incorporating GMDs of the first gel into LMDs, thereby forming composite GMDs with distinct non-biological regions, in this case composite GMDs with one or more liquid regions.

Still another general method for producing GMDs having a plurality of non-biological regions, wherein at least one non-biological region has different properties than at least one other non-biological region, involves the following general steps: (a) forming GMDs of a first gel capable of liquification; (b) suspending said GMDs in a material capable of forming a second gel, (c) incorporating GMDs of said first gel into GMDs of said second gel, and (d) liquifying the first gel, thereby forming GMDs with distinct non-biological liquid regions, in this case also with at least one liquid region.

In order to use the gel microdroplets of this invention in processes involving measurement and/or isolation of biological entities, the composite GMDs should be formed from a suspension or solution which contains the appropriate biological entities, and therefore which contains biological material The composite GMDs of this invention are useful in cases wherein the biological material is composition of biological entities such as small multicellular organisms, groups of cells, individual cells, protoplasts, vesicles, spores, organelles, parasites, viruses, nucleic acid molecules, antibody molecules, antigen molecules, and aggregates of molecules, and is particularly useful in cases wherein the cells are selected from the group consisting of animal cells, plant cells, insect cells, bacterial cells, yeast cells, fungi cells and mold cells,
or are selected from the group consisting of normal human cells, human cancer cells, pathogenic bacteria, pathogenic yeast, mycoplasms, parasites, and pathogenic viruses.

Force coupling entities such as beads, non-biological particles, bubbles, and non-aqueous fluid inclusions with force coupling properties selected from the group consisting of ferromagnetic properties, diamagnetic properties, paramagnetic properties, dielectric properties, electrical charge properties, electrophoresis properties, and optical pressure properties can be also incorporated into one or more gel regions, or liquid regions, in order to provide means for physical manipulation of composite GMDs. Thus, it is useful to provide composite GMDs which contain one or more regions with contain force-coupling entities such as beads, non-biological particles, bubbles, and non-aqueous fluid inclusions with force coupling properties.

The invention also includes extension of the basic process to the creation of composite GMDs comprising more than two distinguishable gel regions. For example, composite GMDs which are GMD/GMDs can be used in a GMD formation process to form GMD/GMD/GMDs, that is, composite GMDs with three distinguishable gel regions.

GMDs containing more than one non-biological gel region can be formed, such that composite GMDs are thereby formed, wherein said composite GMDs are comprised of regions of different gel material, and/or of the same gel material but with different entrapped or bound entities. Thus, for example, a GMD which is a composite GMD made from two different gel materials can contain a first inner region which is comprised of a soft, low density gel such as 0.5% agarose, and a second outer region which is comprised of a harder, higher density gel such as 4% agarose. Continuing this illustration, the 0.5% agarose first inner region can support the growth of cells with less compressive force on the cells, while the second outer region can better confine the growing cells.

GMDs containing at least one liquid region can also be formed. An exemplary process for formation of GMDs wherein a gel material region surrounds a liquid region is as follows. A first step comprises using a process to form GMDs from a gel material capable of subsequent liquification, a second step comprises formation of GMDs which consist of a second gel region completely surrounding the first GMDs, such that the second gel material is different from the first gel material, and is capable of remaining a gel under conditions which liquify the first gel, and a third step comprises liquification of the first gel material, with the result that GMDs with a liquid region surrounded by a gel region are thereby formed. A more specific illustration of this process is as follows. The first step comprises forming liquid microdroplets which contain sodium alginate by forcing a suspension of biological entities with sodium aliginate through a vibrating orifice, thereby breaking up the resulting liquid jet which contains both biological entities and sodium alginate, allowing the resulting liquid microdroplets to enter an aqueous medium containing calcium ions, thereby forming calcium alginate GMDs. The second step comprises concentrating the calcium alginate GMDs by means such as filtration and centrifugation, adding molten agarose at about 37°C, following which the calcium alginate GMD suspension is dispersed into mineral oil, and cooling the dispersion, thereby forming agarose GMDs which contain alginate GMDs, The third step comprises exposing said composite GMDs to an aqueous solution containing sodium chloride and essentially zero calcium, such that sodium and calcium ions can be exchanged, and thereby liquifying the calcium alginate within the composite GMDs.

For convenience it is useful to term GMDs comprised of more than one gel and liquid region as composite GMDs, and to use notation such that GMD/LMDs refers to GMDs formed with a first formed region which is liquid and a second formed region which is gel, and GMD/GMD refers to GMDs formed with a first region which is gel and a second formed region which is gel. Thus, in the preceeding more specific illustration, upon completion of the second step GMDs comprising GMD(agarose)/GMDs(calcium alginate) are formed, and upon completion of the third step GMDs comprising GMD(agarose)/LMDs(sodium alginate) are formed.

### Manipulation of Microdroplets Surrounded by Non-Aqueous Fluids

It is generally desirable to provide means for applying forces on MDs, so as to provide means for manipulating MDs, such as changing or maintaining the locations of MDs, so that external influence from physical, chemical and biological sources can be provided, for purposes such as measurement, incubation and isolation. It is useful to distinguish two classes of processes for applying forces which accomplish changing or maintaining positions of MDs. These two classes are forces which depend on MDs being surrounded by a non-aqueous fluid, and forces which depend on MD force coupling entities, or intrinsic properties of MDs, which are surrounded either by a non-aqueous fluid or an aqueous fluid.

In the case of MDs which are GMDs, following GMD creation in a non-aqueous fluid, the GMDs are left suspended, allowed to settle, or captured on a grid or filter, while surrounded by the non-aqueous fluid, such that the GMDs can now be manipulated through the use of physiochemical interactions that exploit differences in properties of the aqueous GMD interior fluid and the GMD-surrounding non-aqueous fluid.

Alternatively, if GMDs are created in an aqueous fluid, the GMDs can be transfered to a non-aqueous fluid, wherein the GMDs are manipulated by the use of physiochemical interactions that exploit differences in properties of the aqueous GMD interior fluid and the GMD-surrounding non-aqueous fluid.

In this invention MDs can be surrounded by non-aqueous fluids in order to provide a non-aqueous environment which surrounds or suspends microdroplets Representative suitable non-aqueous fluids include liquid hydrocarbons, mineral oils, silicone fluids, ferrofluids, and heavy alcohols. This invention further involves physical manipulation of microdroplets surrounded by a non-aqueous fluid so as to change the position of such microdroplets by applying one or more physical forces, which are due to differences in properties of the MDs and the surrounding non-aqueous fluid.

More particularly, such forces can be applied by selecting forces selected from the group consisting of electrical, magnetic, acoustic and optical forces. While most of these forces are extremely well known in general, the term optical pressure force as applied to particles the size of MDs is more recent (see, for example, Ashkin et al, Nature 330: 769-771, 1987). In the process of this invention it is preferred to utilize electrical force selected from the group consisting of electrophoresis force, iontophoresis force, electrokinetic force, dielectric force and coulombic force, which are well known forces which can be applied to entities with electrical charge and/or dielectric properties which differ from the dielectric properties of the medium surrounding the entities.

A particular process involving such electrical force is carried out using the following steps: (a) providing a non-aqueous fluid environment, (b) providing a plurality of charged electrodes within said non-aqueous fluid environment wherein at least two electrodes are of opposite polarity, (c) injecting an electically charged material capable of forming electrically charged microdroplets into the non-aqueous fluid; and (d) moving one or more charged microdroplets by means of an electrical force associated with potential differences which are applied between two or more electrodes, such that it is possible to produce microdroplets within a non-aqueous fluid, to introduce microdroplets into a non-aqueous fluid, to move microdroplets within a non-aqueous fluid, and to remove microdroplets from a non-aqueous fluid. Alternatively, MDs formed by any means and surrounded by a low electrical conductivity medium can be charged by contacting the MDs with a charged electrode.

It is thus possible to use such forces to manipulate the position of MDs with respect to measurement entities such as light emitting diodes, lasers, electrical capacitors, electrical inductors, electrical resistors, thermistors, thermocouples, fiber optics, photodiodes, phototransistors, photocells, piezoelectric sensors, specific ion electrodes, oxygen electrodes, carbon dioxide electrodes, pH electrodes and electrodes allowing dielectric property measurement. More specifically, such physical force is utilized to move MDs into proximity to such measurement entities, to maintain MDs in proximity to such measurement entities, and to remove MDs away from such measurement entities.

In addition to physically manipulating MDs with respect to one or more such measurement entities, in the process of this invention physical force is also utilized to move MDs which are located within measurement apparatus such as light measuring instruments, light microscopes, fluorescence microscopes, luminometers, fluorometers, photometers, time-resolved fluorometers, image analysis systems, colorimeters, spectrofluorimeters, particle counters, particle measuring systems, photoacoustic instruments, acoustic absorption instruments, acoustic microscopes, dielectric spectrometers, electrical impedance measuring instruments, calorimeters, thermal property measurement instruments and piezoelectric mass loading instruments.

Similarly, the process of this invention can be used to cause MDs to come into contact with other MDs, or for contacted MDs to be separated. The former is particularly useful to enhancing collisions and coalescence of LMDs, while the latter is particularly useful for separating weakly adhering GMDs.

Likewise, the process of applying one or more forces to MDs surrounded by a non-aqueous fluid can be used to physical force is used to provide physical manipulations of microdroplets selected from the group consisting of moving microdroplets into proximity of, maintaining microdroplets in the proximity of, and removing microdroplets away from so that such MDs are exposed to external sources of physical influence, or exposed to sources of chemical influence. Such MDs surrounded by a non-aqueous fluid can be exposed to any physical source of external influence, including sources of heat, electric fields, magnetic fields, electromagnetic radiation, optical radiation, ionizing radiation and acoustic radiation. Suitable sources of chemical influence include those which produce, release, modify or consume chemical compounds which are capable of dissolving in both the surrounding non-aqueous fluid and the aqueous interior of such MDs.

Finally, all of such manipulations are of particular utility in the case that at least one of the microdroplets contains at least one biological entity such as small multicellular organisms, groups of cells, individual cells, protoplasts, vesicles, spores, organelles, parasites, viruses, nucleic acid molecules, antibody molecules, antigen molecules, and aggregates of molecules.

### Provision of External Influence on Biological Entities

In many tests and assays the interaction of biological entities with external sources of chemicals, biological factors or physical fields are extremely important, and it is highly desirable to provide means for extending MD measurement and isolation processes to include the effects of such influence. In order to provide such external influence on biological entities, the biological entities are incorporated into MDs , so that MDs which contain biological entities can be introduced into position of proximity to a source, maintained in position in proximity to a source or removed from proximity to a source. In the general practice of this invention, sources of influence include biological sources of influence, chemical sources of influence, physical sources of influence, and combinations of biological, chemical and physical sources of influence are provided by using MDs.

In general, however, it is often preferred to provide influence by utilizing MDs which are GMDs, particularly GMDs which are surrounded by an aqueous medium such that the aqueous medium contacts the aqueous interior medium of GMDs, thereby allowing chemical and small biological entities to be exchanged between the GMDs and the aqueous medium. It is also possible to provide influence by using LMDs and/or GMDs surrounded by a non-aqueous fluid, as most types of physical influence is readily provided, but the chemicals which can be exchanged is more limited, and relatively few biological entities can be exchanged. For aqueous surrounded GMDs, exemplary surrounding aqueous media include aqueous growth medium, physiological fluids, human body fluids, animal body fluids, organ perfusates, suspensions of cells, suspension of small multicellular organisms, animal tissue homogenates, plant tissue homogenates, cell culture medium, culture medium for microorganisms containing biological material, defined culture medium for microorganisms, defined culture medium for mammalian cells, blood, blood plasma, urine, cerebral spinal fluid and interstitial fluid. Upon exposure of GMDs to one or more such aqueous media or environments, and depending on the molecular filteration characteristics of the gel matrix, most chemical compounds and some small biological entities can enter the GMD by partitioning into the aqueous environment of the gel matrix. These can be transported, often by diffusion, but also by drift and/or convection, within the GMD, thereby exposing biological entities contained within GMDs to chemical compounds and/or small biological entities contained within the aqueous environment.

In the case of such GMDs, the present invention provides means for influencing biological entities to external influence though the general means of providing physical forces which allow manipulation of GMDs, such that GMDs can be moved into and within an aqueous environment. Such manipulation of GMDs allows GMDs to be positioned in proximity to sources of influence which are external to GMDs, such that chemical compounds of the aqueous environment in proximity to one or more external sources can enter GMDs and thereby interact with biological entities within GMDs, and thus provide influence on the biological entites. More specifically, this process provides a general means for providing external influence on biological entities by utilizing physical manipulation of GMDs, containing at least one biological entity, so as to affect the proximity of at least one GMD to an influence source which is external to the GMDs. Thus, it is particulalry useful to provide influence on biological entities by utilizing GMDs in an aqueous fluid.

However, because of the greater generality of this invention, it is appropriate to describe most of this invention in terms of the use of the more general case of MDs surrounded by either aqueous fluids or non-aqueous fluids. Following the provision of influence, it is generally useful to determine the effect of the influence on biological properies of biological entities contained within the MD. This is particularly relevant for biological properties such as biological material, biochemical activity, production of molecules, degradation of molecules, secretion of molecules, metabolism, membrane integrity, enzyme activity and growth, as these are directly or indirectly involved in many assays and tests In order to flexibly provide influence due to one or more biological sources it is useful to provide such biological influence by selecting a source from the group consisting of cells, feeder cells, animal tissues, plant tissues, animal tissue homogenates, plant tissue homogenates, perfused organs, whole animals, plants, cell suspensions, biological entities within gel microdroplets, cell cultures, body fluids.

As used herein, the term biological influence describes the influence associated with chemical compounds and small biological agents such as phage, which have biological origin and can be present in the aqueous environment in proximity to a biological source. Representative biological sources include cells, feeder cells, animal tissues, plant tissues, animal tissue homogenates, plant tissue homogenates, perfused organs, whole animals, plants, cell suspensions, biological entities within gel microdroplets, cell cultures, body fluids. Chemical sources include any entity which provides to an aqueous environment one or more chemical compounds, and can therefore include biological sources. Physical sources include any entity which is the source of physical conditions in an aqueous environment. These include physical processes such heat, electrical energy, magnetic energy, optical energy, radioactivity or ionizing radiation, and acoustic energy which alter chemical composition of chemical compounds in an aqueous environment. Physical sources also include any entity which directly provides heat, electrical energy, magnetic energy, optical energy, radioactivity or acoustic energy to MDs, thereby providing influence on biological entities contained within MDs.

By using the process of this invention in which MDs can be manipulated, including specifically manipulation which results in MDs being near, maintained near and removed from one or more sources of external influence, external influence can be caused on biological entities such as small multicellular organisms, groups of cells, individual cells, protoplasts, vesicles, spores, organelles, parasites, viruses, nucleic acid molecules, antibody molecules, antigen molecules, and aggregates of molecules It is particularly useful to provide influence on cells such as animal cells, plant cells, insect cells, bacterial cells, yeast cells, fungi cells and mold cells , and such as normal human cells, human cancer cells, pathogenic bacteria, pathogenic yeast, mycoplasms, parasites, and pathogenic viruses In the important case of mammalian cells it is preferred to provide influence on cells such as of normal human cells, cancerous human cells and hybridoma cells

In order to provide complex biological influence, wherein many different and complex biochemical pathways may be involved, and wherein different tissues may participate, it is possible to reversibly position MDs, particularly GMDs, within whole animals such as mouse, immunodeficient mouse, rat, rabbit, primate, goat, dog, horse, pig, guinea pig and and human being. These are important biological sources of external influence, and additional related sources comprise one or more perfused organs from such animals Thus, for example, GMDs containing magnetic force-coupling entities in addition to biological entities can be inserted into an animal, so that complex biological influence can be provided. If desired, the biological entities within the GMDs can be protected from an immune response by the animal by providing an appropriate molecular weight cutoff property for the gel matrix of the GMDs. An exemplary means for accomplishing this protection is to use composite GMDs wherein an outermost gel region surrounding one or more biological entity-containing regions is used with a gel with a moderately low molecular weight cutoff.

Additional flexibility can be provided by supplying influence due to one or more sources which are themselves contained within MDs, and in this general case it is the relative position of the influencing MDs and the influenced MDs which is important. Thus either or both types of MDs can be manipulated The flexibility of this invention can also be increased by providing and utilizing gel material for MDs which are GMDs wherein the gel matrix which allows selective passage of molecules

In order to physically manipulate MDs with respect to one or more sources of influence, the process of this invention provides physical forces such as electrical force, magnetic force, acoustic force, optical pressure force, wherein the means for generation of such forces is well-known or is described elsewhere in this disclosure In order to more effectively provide such forces it is useful to supply one or more types of coupling entities, or force-coupling entities, wherein such entities are selected from the group consisting of beads, non-biological particles, bubbles, and non-aqueous fluid inclusions with force coupling properties selected from the group consisting of ferromagnetic properties, diamagnetic properties, paramagnetic properties, dielectric properties, electrical charge properties, electrophoresis properties, and optical pressure properties It is generally preferred to provide magnetic force by incorporating magnetic coupling entities into MDs , and an exemplary type of magnetic force coupling entities are those comprised of magnetite

An illustration of a source of physical influence relates to manipulations and measurements involving the use of heat and ionizing radiation for treatment of cancer, wherein cancer cells can be killed by application of heat and/or ionizing radiation. The use of ionizing radiation is well established as a therapeutic modality for the treatment of cancer, and the use of elevated temperature, or hyperthermia, either alone or in combination with ionizing radiation, has been more recently established as a therapeutic modality. In such treaments, a general tendency of increased cancer cell killing relative to normal cell killing is exploited. In order to carry out *in vitro* tests of the sensititivy, and the variability of sensitivity, of cancer cells to heat and/or ionizing radiation, cancer cells can be incorporated into GMDs. The GMDs are then exposed to the physical influence comprising elevated temperature and/or exposure to ionizing radiation, and then, if desired, incubated. The MDs are then measured for changes in the amount of biological material or biochemical activity. It is preferred to measure the amount of biological material relative to one or more controls, thereby providing a measure of growth of the cells exposed to physical influence relative to the growth of control cells in which are not exposed to the physical influence. Further, staining protocols can be used which test for membrane integrity. Thus, the fluorescent stain propidium iodide, and additional or separate staining with vital stains such as FITC diacetate can be used as the basis of a short term indicator of viability.

The process of this invention relating to provision of physical influence can also be carried out under conditions that approximate *in vivo* conditions. For example, GMDs containing cells to be tested can be placed within the body of an animal, such as a immunocompromised mouse, and the animal then exposed to conditions which approximate hyperthermia and/or ionizing radiation treatment in animal species, including humans. This provides external influence on the cells which is a combination of physical influence relating to heat and/or ionizing radiation, and also external biological influence relating to the biochemical environment within the so treated animal. Following such provision of external influence on cells, measurements such as those relating to cell death, cell survival, cell growth and biochemical activity can be carried out using aspects of this invention described elsewhere in the present disclosure. Thus, exposure to heat influence can be provided and utilized to determine cell death and cell growth under conditions relating to cancer hyperthermia treatment.

Similarly, the effect of the physical influence of ionizing radiation on biological entities, particularly normal mammalian cells and cancerous mammalian cells, can be tested by the process of this invention, either under completely *in vitro* conditions, or, by inserting MDs, particuarly GMDs, into a test animal, or even a human being, under essentially *in vivo* conditions. Thus, this invention can be used to determine cell death and cell growth under conditions relating to cancer radiation treatment.

The process of this invention can also be applied to combinations of cancer treatment which include sequential or simultaneous combinations of physical treatment such as ionizing radiation and hyperthermia, and chemical treatment, including established chemotherapy and newer therapy based on monoclonal antibodies and the like.

One or more sources of external influence can be selected from the group consisting of mammalian cells, yeast cells and bacterial cells. In the case of mammalian cell culture, biological entities such as cultured mammalian cells can be exposed to "feeder cells". These "feeder cells" comprise the external source of influence, which in this case, are compounds which enhance the growth of cells. In addition, it is often preferred to influence biological entities, particularly cells, to the complex biochemical influence which is be provided by whole animals, especially animals selected from the group consisting of mouse, immunodeficient mouse, rat, rabbit, primate, goat, dog, horse and and human being. For example, the complex metabolic processes of whole animals can provide activation and degredation of chemical compounds which is generally difficult to duplicate in cell culture. Thus, the use of the present invention provides a general means for reversibly exposing biological entities such as cells to complex external influence resulting from whole animal activation and metabolism of chemical compounds.

One or more sources of external influence can be incorporated into microdroplets, thereby providing a means for influencing biological entities wherein the source of influence can be manipulated by altering the position of the source with respect to the influenced biological entities which are in other MDs In this general case, different types of force can be used with the influencing MDs, for example magnetic force, while another force, for example, a force depenedent on the mass density of MDs, can be used with the influenced MDs Sources of influence can be incorporated into MDs, and the resulting MDs manipulated so as to provide influence on biological entities which are not contained in MDs For example, feeder cells can be incoporated into GMDs with are also provided with magnetic force coupling entities such as magnetite, so that the feeder cells can be positioned in proximity to cultured cells which are not in GMDs, and, following any desired incubation, the GMDs containing the feeder cells can be readily removed.

Forces suitable for manipulation of MDs surrounded by a non-aqueous fluid are described elsewhere in this disclosure. Any of a variety of physical forces can be used to introduce GMDs surrounded by an aqueous fluid into close proximity to a source of influence, maintain GMDs in close proximity to a source of influence, and to remove GMDs from close proximity to a source of influence.

Electrical force can be provided by interaction of an applied electric field, including an electric field with field gradients, with dielectric particles provided within the gel matrix of GMDs, or more generally by interaction with charge groups associated with the gel matrix or coupling entities provided within GMDs. Magnetic force can by applied by providing coupling entities within GMDs, said coupling entities having diamagnetic, paramagnetic or ferromagnetic properties different from the aqueous medium which surrounds the GMDs. Representative coupling entities for applying a magnetic force are magnetic particles, magnetic granuals, ferrofluid inclusions and the like. A preferred embodiment comprises magnetite (*Fe*₂*O*₄) particles within GMDs. Alternatively, magnetic force can be applied directly in those cases wherein the diamagnetic, paramagnetic, ferromagnetic or electrical conductivity properties of GMDs differs from the diamagnetic, paramagnetic, ferromagnetic or electrical conductivity properties of the aqueous medium which surrounds the GMDs. Acoustic forces can be applied by applying sound or acoustic fields which preferentially interacts with the gel matrix of GMDs, or with coupling entities contained within GMDs, such that the inclusion of said coupling entities results in GMDs having a different mass density, or different mechanical compliance, than the aqueous medium which surrounds the GMDs. Optical pressure force can be applied by utilizing optical radiation which interacts with the gel matrix or coupling entities contained within the GMDs, or with the larger biological entities contained within the GMDs (see, for example, Ashkin et al, Nature 330: 769 - 771, 1987).

After one or more exposures to one or more compounds, and following one or more incubation periods with or without controls, the MDs, that is LMDs and GMDs, are exposed to one or more staining processes, which are described elsewhere in this application, but, as also described elsewhere, if naturally occuring signals are adquate, the staining processes are omitted.

Following exposure of the GMDs to suitable stains, individual GMDs are individually measured, or measured in small groups, provided that the probability of finding more than one cell-containing GMD in the group is small. In the exemplary case of cells stained by fluorescent compounds, optical analysis such as digital fluorescence microscopy or flow cytometry is used to analyze individual GMDs, using a wavelength band sufficiently different from that used for any detection of measurement of GMD properties so that simultaneous, or serial, measurement of GMD properties and of cells with said GMD are possible. The associated fluorescence signals are acquired and analyzed, with correction for spectral overlap if necessary, by conventional means.

The magnitude of the optical signal due to the cell stain in each GMD, or small group of GMDs, is compared to the fluorescence of individual cells, whether or not such individual cells are entrapped in GMDs, thereby providing a calibration. Comparison of the GMD signal magnitude to that of individual cells provides the basis for determination of growth of individual cells, for which the growth determination can often be made within about one generation time, but without a need for significant prior culture to obtain large numbers of cells, and growth can also be determined over several generations if desired.

By making a large number of such individual cell growth determinations, the distribution of growth rate, distribution of lag time, and the plating efficiency caused by the exposure to one or more compounds or agents can be automatically determined by computer calculation. Other measurements relating to cell survival and cell death, particularly vital stains such as transmembrane potential stains, membrane exclusion stains and intracellular enzyme activity responsive stains, can also be used. Manual or visual inspection and scoring of GMDs can also be used, but is relatively labor intensive and therefore more prone to error. Thus, the preferred process is that conducted using the automated measurement means.

Similarly, measurements, assays, tests and isolation procedures directed towards the use of biological entities for the production of desirable compounds, or the use of biological entities to provide processes directed towards the degredation or modifcation of undesirable compounds, such as toxic wastes, can benefit from biological or biochemical activation of the biological entities, which activation comprises a form of influence on the biological entities.

### Example I Manipulation of Charged GMDs Surrounded by Mineral Oil by electrical force

Agarose GMDs were created by injecting an aqueous fluid containing molton agarose (Sigma Type IX) and 50µmolar FITC-dextran ("Green" fluorescence") through a 21 gauge stainless steel needle into a vortexed Falcon 2063 polypropylene test tube, the outer surface of which was covered with aluminum foil, so that an electrical capacitance occurred between the stainless steel needle and the aluminum foil. A variable high voltage power supply (Kepco Model HB-20M) was connected with one terminal to the stainless steel needle and the other to the aluminum foil, so that upon applying an electrical potential while injecting the molton agarose solution the resulting liquid microdroplets was electrically charged. The combined action of vortexing of the mineral oil and the electrical charging of the the aqueous liquid both served together to disperse the aqueous liquid into electrically charged microdroplets within the non-aqueous mineral oil. GMDs were then created by transient cooling, such that the resulting GMDs could also be charged, depending upon the electrical resistivity of the particular grade of mineral oil used, as the well-known charge relaxation time is governed by both the dielectric constant of the oil and the resistivity of the oil.

Following such GMD formation, a small volume of mineral oil containing GMDs was transfered to a parallel plate capacitor/observing chamber wherein potentials could be applied to the two parallel electrodes, and the motion of fluorescein-containing GMDs observed by fluoresence microscopy as various potentials were applied to the two electrodes. In this example wherein an ordinary mineral oil was used, the results were equivocal, as the charge relaxation time was estimated to be 30 seconds, which predicts that the GMDs would not remain charged for a long enough time to be observed in these experiments.

Manipulation of the GMDs is now attempted by applying electrical potentials to electrodes bathed in the mineral oil, so that a force can be applied on GMDs by virtue of their electrical charge. Generally, the following are observed. GMDs near an electrode which is charged to a potential causing electrode surface charge opposite in sign to the charge on the GMD is attracted towards the electrode, such that the movement of such a GMD can be observed using a microscope. Changing the electrode potential to the same magnitude but opposite polarity results in electrode surface charge of opposite sign, such that the same GMD is now repelled from the electrode. It was concluded that water introduction into the mineral oil had likely occurred, and that therefore the observed movement of GMDs in response to the applied electrode potentials was due to electrophoresis.

## Claims

1. A method of micro-manipulation comprising the steps of:
a) forming an aqueous microdroplet which contains the subject of the micromanipulation and a force coupling component having ferromagnetic, diamagnetic, paramagnetic, dielectric, electrical charge, electrophoretic and/or optical pressure properties,
b) surrounding the microdroplet with a non-aqueous medium having ferromagnetic, diamagnetic, paramagnetic, dielectric, electrical charge, electrophoretic and/or optical pressure properties which differ from those of the force coupling component, and
c) applying a physical force selected from electrical, magnetic, acoustic and optical forces whereby a differential response to the applied force of the force coupling component relative to the non-aqueous medium causes micro-manipulation of the subject of the micromanipulation, the subject of the micromanipulation comprising a cell, vesicle, spore, organelle, virus, nucleic acid molecule, antibody molecule or antigen molecule.

2. A method according to Claim 1 wherein the aqueous microdroplet is a liquid, gel or gel/liquid composite.

3. A method according to Claim 1 or 2 wherein the force coupling component comprises a gel.

4. A method according to any of the preceding claims wherein the force coupling component comprises beads, non-biological particles, bubbles and/or non-aqueous fluid inclusions.

5. A method according to any one of the preceding claims wherein at least two aqueous microdroplets are formed and the applied physical force causes aggregation of the aqueous microdroplets

6. A method according to any one of the preceding claims wherein at least two aqueous microdroplets are formed and the applied physical force causes dispersion of the aqueous microdroplets.

7. A method for detecting the manipulation of an aqueous microdroplet by an applied physical force relative to a non-aqueous medium in which the microdroplet is suspended, comprising the steps of
a) manipulating an aqueous microdroplet according to the method of claim 1; and
b) detecting the differential response of the aqueous microdroplet relative to the non-aqueous medium.

8. A method according to Claim 7 further comprising the step of forming an aqueous solution including a solute which causes the aqueous solution to be differentially responsive to the applied physical force relative to said non-aqueous medium and wherein the aqueous solution is used to form at least one aqueous microdroplet whereby the aqueous microdroplet is differentially responsive to the applied physical force relative to the non-aqueous medium.

## Patentansprüche

1. Mikromanipulationsmethode, die folgende Schritte aufweist:
a) Bildung eines wäßrigen Mikrotröpfchens, das den Gegenstand der Mikromanipulation enthält sowie eine kräftekoppelnde Komponente mit eisenmagnetischer, diamagnetrischer, paramagnetischer, dielektrischer, elektrischer Ladung, elektrophoretischen und/oder optischen Druckeigenschaften,
b) Umgeben des Mikrotröpfchens mit einem nichtwäßrigen Medium mit eisenmagnetischer, diamagnetischer, paramagnetischer, dielektrischer, elektrischer Ladung, elektrophoretischen und/oder optischen Druckeigenschaften, die sich von denen der kräftekoppelnden Komponente unterscheiden,
c) Anwenden einer physikalischen Kraft, ausgewählt aus elektrischen, magnetischen, akustischen und optischen Kräften, wodurch eine Differenzierungsreaktion auf die ausgeübte Kraft der kräftekoppelnden Komponente, bezogen auf das nichtwäßrige Medium, die Mikromanipulation des Mikromanipulationsgegenstandes bewirkt, wobei der Gegenstand der Mikromanipulation eine Zelle, ein Vesikel, eine Spore, eine Organelle, ein Virus, ein Nukleinsäuremolekul, ein Antikörpermolekül oder ein Antigenmolekül umfaßt.

2. Methode gemäß Anspruch 1, dadurch gekennzeichnet, daß das wäßrige Mikrotröpfchen eine Flüssigkeit, ein Gel oder eine Zusammensetzung aus einem Gel und einer Flüssigkeit ist.

3. Methode gemäß Anspruch 1 oder 2, dadurch gekennzeichnet, daß die kräftekoppelnde Komponente ein Gel enthält.

4. Methode nach einem der vorhergehenden Ansprüche, wobei die kräftekoppelnde Komponente Kügelchen, nichtbiologische Partikel, Bläschen und/oder nichtwäßrige Flüssigkeitseinschlüsse enthält.

5. Methode nach einem der vorhergehenden Ansprüche, wobei mindestens zwei wäßrige Mikrotröpfchen gebildet werden und die angewandte physikalische Kraft die Zusammenballung der wäßrigen Mikrotröpfchen bewirkt.

6. Methode nach einem der vorhergehenden Ansprüche, wobei mindestens zwei wäßrige Mikrotröpfchen gebildet werden und die angewandte physikalische Kraft die Dispersion der wäßrigen Mikrotröpfchen bewirkt.

7. Methode zur Aufdeckung der Manipulation eines wäßrigen Mikrotröpfchens durch eine angewandte physikalische Kraft, bezogen auf ein nichtwäßriges Medium, in dem das Mikrotröpfchen suspendiert ist, die folgende Schritte umfaßt:
a) Manipulation eines wäßrigen Mikrotröpfchens gemäß der in Anspruch 1 beschriebenen Methode und
b) Aufdecken der Differenzierungsreaktion des wäßrigen Mikrotröpfchens, bezogen auf das nichtwäßrige Medium

8. Methode gemäß Anspruch 7, die als weiteren Schritt die Bildung einer wäßrigen Lösung aufweist, die einen gelösten Stoff enthält, durch den die wäßrige Lösung mit einer Differenzierung auf die angewandte physikalische Kraft, bezogen auf das nichtwäßrige Medium, reagiert und wobei die wäßrige Lösung dazu verwendet wird, mindestens ein wäßriges Mikrotröpfchen zu bilden, wodurch das wäßrige Mikrotröpfchen durch Differenzierung auf die angewandte physikalische Kraft in bezug auf das nichtwäßrige Medium reagiert.

## Revendications

1. Un procédé de micromanipulation comprenant les étapes de:
a) former une microgouttelette aqueuse qui contient le sujet de la micromanipulation et un élément de couplage de forces ayant des propriétés ferromagnétiques, diamagnétiques, paramagnétiques, diélectriques, de charge électrique, électrophorétiques et/ou de pression optique,
b) entourer la microgouttelette avec un milieu non-aqueux ayant des propriétés ferromagnétiques, diamagnétiques, paramagnétiques, diélectriques, de charge électrique, électrophorétiques et/ou de pression optique, qui différent de celles de l'élément de couplage de forces, et
c) appliquer une force physique sélectionnée parmi les forces électriques, magnétiques, acoustiques et optiques, au moyen de laquelle une réponse différentielle, à la force appliquée, de l'élément de couplage de forces, par rapport au milieu non-aqueux, provoque une micromanipulation du sujet de la micromanipulation, le sujet de la micromanipulation comprenant une cellule, une vésicule, une spore, un organelle, un virus, une molécule d'acide nucléique, une molécule d'anticorps ou une molécule d'antigène.

2. Un procédé, selon la revendication 1, dans lequel la microgouttelette aqueuse est un liquide, un gel ou un composite gel/liquide.

3. Un procédé, selon les revendications 1 ou 2, dans lequel l'élément de couplage de forces comprend un gel.

4. Un procédé, selon l'une quelconque des revendications précédentes, dans lequel l'élément de couplage de forces comprend des perles, des particules non-biologiques, des bulles et/ou des inclusions d'un fluide non-aqueux.

5. Un procédé, selon l'une quelconque des revendications précédentes, dans lequel au moins deux microgouttelettes aqueuses sont formées et la force physique appliquée provoque l'agrégation des microgouttelettes aqueuses.

6. Un procédé, selon l'une quelconque des revendications précédentes, dans lequel au moins deux microgouttelettes aqueuses sont formées et la force physique appliquée provoque la dispersion des microgouttelettes aqueuses.

7. Un procédé pour détecter la manipulation d'une microgouttelette aqueuse par une force physique appliquée, par rapport au milieu non-aqueux dans lequel la microgouttelette est suspendue, comprenant les étapes de
a) manipuler une microgouttelette aqueuse, conformément au procédé de la revendication 1; et
b) détecter la réponse différentielle de la microgouttelette aqueuse, par rapport au milieu non-aqueux.

8. Un procédé, selon la revendication 7, comprenant, en outre, l'étape de former une solution aqueuse comprenant une soluté qui fasse en sorte que la solution aqueuse soit sensible différentiellement, à la force physique appliquée, par rapport audit milieu non-aqueux et dans lequel la solution aqueuse est utilisée pour former au moins une microgouttelette aqueuse, au moyen de laquelle la microgouttelette aqueuse est sensible différentiellement, à la force physique appliquée, par rapport au milieu non-aqueux.
